# EUROPEAN PATENT SPECIFICATION

(11) **EP 3 759 209 B1**
(45) Date of publication and mention of the grant of the patent: **11.11.2026**
(21) Application number: 19760265.9
(22) Date of filing: 27.02.2019
(51) Int. Cl.: C12M 1/00, C12P 5/02, C12P 7/06, C12N 9/34, C12P 7/10

(54) **GLUCOAMYLASE ENGINEERED YEAST AND FERMENTATION METHODS**
MIT GLUCOAMYLASE MANIPULIERTE HEFE UND GÄRUNGSVERFAHREN
LEVURE MODIFIÉE À GLUCOAMYLASE ET PROCÉDÉS DE FERMENTATION

(30) Priority: 28.02.2018 US 201862636716 P
(43) Date of publication of application: 06.01.2021
(60) Divisional application: 26167171.3 / 4 737 579
(73) Proprietor: Cargill, Incorporated, Wayzata, Minnesota 55391 (US)
(72) Inventor: MILLER, Chris, Andover, Minnesota 55304 (US); RUSH, Brian, Minneapolis, Minnesota 55410 (US); STANTON, Brynne, Boston, Massachusetts 02210 (US); DUNN, Joshua, Boston, Massachusetts 02210 (US)
(74) Representative: Forresters IP LLP
(86) International application number: PCT/US2019/019805
(87) International publication number: WO 2019/168962

(56) References cited:
- WO-A1-01/42482
- WO-A1-2012/019159
- WO-A1-2017/106739
- WO-A1-2018/204798
- WO-A1-2019/191263
- US-A1- 2011 269 185
- US-A1- 2012 045 812
- DATABASE Geneseq [online] 27 December 2018 (2018-12-27), "Rhizopus microsporus glucoamylase, SEQ ID 108.", XP002805075, retrieved from EBI accession no. GSP:BFU53535 Database accession no. BFU53535
- DATABASE UniParc [online] 15 December 2014 (2014-12-15), "Rhizopus microsporus Glucoamylase", XP002805076, accession no. UniParc - UPI00053810D6 Database accession no. UPI00053810D6
- ASHIKARI T ET AL: "RHIZOPUS RAW-STARCH-DEGRADING GLUCOAMYLASE ITS CLONING AND EXPRESSION IN YEAST", AGRICULTURAL AND BIOLOGICAL CHEMISTRY, AGRICULTURAL CHEMICAL SOCIETY OF JAPAN, JP, vol. 50, no. 4, 1 January 1986 (1986-01-01), pages 957 - 964, XP008120586, ISSN: 0002-1369
- DATABASE Geneseq [online] 29 March 2012 (2012-03-29), "Rhizopus oryzae glucoamylase (RhGA), SEQ ID 8.", XP002805077, retrieved from EBI accession no. GSP:AZT47646 Database accession no. AZT47646
- DATABASE Protein [online] 9 April 2015 (2015-04-09), "Putative Glucoamylase (Fragment) [Rhizopus Microsporus", XP055635202, retrieved from NCBI Database accession no. CEG69155.1

## Description

### FIELD OF THE INVENTION

The current invention relates to yeast engineered with exogenous glucoamylase nucleic acids and fermentations methods for producing a bioproduct such as ethanol.

### BACKGROUND

Many fermentation feedstocks are derived from plant sources (e.g., corn mash) where the carbohydrates are predominantly in the form of starch polymers. The starch polymers in such feedstocks must be treated to low molecular weight sugars that can be consumed by the yeast and used for growth and bioproduct production. Typical treatments include acid and/or enzymatic hydrolysis where the polymer chain is hydrolyzed to generate the sugars that can be used by the yeast. Starch degrading enzymes such as alpha amylases and glucoamylases can be added to convert the polymer to simple sugars. However, such enzyme additions can add significant cost and complexity to the fermentation process.

Heterologous expression and functionality of enzymes in yeast to aid in starch hydrolysis can be challenging, as it is difficult to know if the nucleic acid will be expressed properly and a functional enzyme will form, and if an active form of the enzyme will be secreted from the cell. It is also challenging to engineer yeast for growth and bioproduct production at non-optimal conditions, such as high temperatures, and in high bioproduct titers. For example, while ethanol production by fermentation is a well know industrial process, maintaining ethanol rates, titers, and yields while at the same time engineering the yeast to reduce reliance on supplemental enzymes, growth under non-optimal conditions (e.g., temperature), and minimizing by-product formation can be technically difficult. Increased ethanol concentration and accumulation of undesirable byproducts can also be detrimental to cell health.
WO 2018/204798 A1 relates to genetically engineered yeasts having a heterologous trehalase gene and fermentation processes for using such yeasts.
*"*Rhizopus microsporus Glucoamylase", UniParc, (20141215), Database accession no. UPI00053810D6, is a database entry disclosing a *Rhizopus microsporous* glucoamylase sequence.
Ashikari T et al, "Rhizopus raw-starch-degrading glucoamylase its cloning and expression in yeast", Agricultural and Biological Chemistry, Agricultural Chemical Society of Japan, JP, (19860101), vol. 50, no. 4, ISSN 0002-1369, pages 957 - 964, discloses a glucoamylase gene that has been cloned from a *Rhizopus* genomic DNA library using synthetic oligonucleotides corresponding to the amino acid sequence of the glucoamylase.

### SUMMARY OF THE INVENTION

The invention is set out in the appended set of claims. The invention relates to engineered yeast and fermentation methods, wherein the engineered yeast are able to secrete a heterologous glucoamylase (GA) into a fermentation medium and provide glucoamylase activity (E.C. 3.2.1.3) on a fermentation substrate. The invention also relates to methods for producing bio-derived products, such as ethanol, via fermentation using the engineered yeast.

In one aspect, experimental studies associated with the current application identified fungal glucoamylase genes that, when introduced exogenously into yeast, allowed it to grow well on feedstocks containing low glucose and high starch amounts, and produce high levels of bioproduct. The results indicated that the engineered yeast were able to secrete glucoamylase into the fermentation medium and that the glucoamylase was enzymatically active towards the starch to generate sufficient glucose for growth and bioproduct production. Other benefits associated with the disclosure include improved fermentation and bioproduct production at elevated fermentation temperatures. Yet other benefits associated with the disclosure include reduced amounts of glucose at the end of the fermentation period.

The claimed invention provides an engineered yeast comprising an exogenous nucleic acid encoding a glucoamylase comprising a sequence having 81% or greater sequence identity to SEQ ID NO: 1 (*Rhizopus microsporus* glucoamylase), wherein the yeast is capable of producing ethanol at a rate of 1 g/L*h or greater during a fermentation process, and wherein there are 2-8 copies of the exogenous nucleic acid in the cell. In an embodiment, the engineered yeast is capable of producing ethanol at a rate of 1.0, 1.5, 2.0, 2.5, 3.0, 3.5, 4.0, 4.5, or 5.0 g/L*h or greater during a fermentation process. The claimed invention also provides an engineered yeast comprising an exogenous nucleic acid encoding a glucoamylase comprising a sequence having 81% or greater sequence identity to SEQ ID NO: 1 (*Rhizopus microsporus* glucoamylase), wherein the yeast is capable of producing ethanol at a rate of 1 g/L*h or greater during a fermentation process, and wherein the genetically engineered yeast does not express a heterologous trehalase. In another embodiment, the engineered yeast is capable of producing (a) at least 70g/kg of ethanol in a fermentation medium made from a glucose polymer-containing feedstock having (i) a DE of not greater than 50. In another embodiment, the engineered yeast is capable of producing (a) 90 g/kg or greater, 120 g/kg or greater, 130 g/kg or greater, or 140 g/kg or greater of ethanol in a fermentation medium made from a glucose polymer-containing feedstock having (i) a DE of about 30. In embodiments, the amount of 70g/kg of ethanol may be produced within 48 hours of inoculation in a fermentation medium with the feedstock. In embodiments, the glucose concentration may not be greater than 5% (wt) in the fermentation medium at the beginning (inoculation) of the fermentation process. In embodiments, the feedstock may provide an amount of glucose polymer-containing feedstock sufficient to produce 70g/kg ethanol, for example about 20 wt% glucose-polymer feedstock in the medium. In embodiments, the feedstock may have one or more of the following properties: the feedstock is a starch hydrolysate; the glucose-polymer has predominantly α-1,4 linkages; the feedstock substantially excludes cellulosic materials (e.g., less than 20%, 15%, or 10% of cellulosic material).

In an embodiment, the yeast is capable of producing (a) at least 70g/kg of ethanol in a fermentation medium made from corn mash having a DE of 30 ± 2, wherein the fermentation medium comprises about 32% dry weight of corn, and a pH 5.8, 35 ppm CaCl, 1900 ppm urea, 5 ppm ampicillin, wherein the staring yeast concentration is 0.1 (OD600), and fermentation is carried out at 48 hrs at 30°C with agitation. The engineered yeast comprise a glucoamylase having 81%, 82%, 83%, 84%, 85%, 90%, 95%, 96%, 97%, 98%, or 99% or greater sequence identity to SEQ ID NO:1.

The claimed invention also provides a fermentation method for producing a bioproduct, comprising forming a fermentation medium from a glucose polymer-containing feedstock, and then fermenting the fermentation medium using an engineered yeast comprising an exogenous nucleic acid encoding a glucoamylase comprising a sequence having 81% or greater sequence identity to SEQ ID NO:1, wherein there are 2-8 copies of the exogenous nucleic acid in the cell. Fermentation is carried out over a period to produces the bioproduct, such as ethanol.

In more specific embodiments, ethanol is produced during the fermentation period to an amount of 70 g/kg or greater, 90 g/kg or greater, 120 g/kg or greater, 130 g/kg or greater, or 140 g/kg or greater, in the fermentation medium. In some embodiments, in the fermentation method, the glucose polymer-containing feedstock has a dextrose equivalent (DE) that is of about 50 or less. In some embodiments, glucose is not greater than 5 wt % of solids materials in the feedstock. Optionally, supplemental glucoamylase can be introduced into the fermentation methods to increase bioproduct titers.

In some embodiments using the engineered yeast with glucoamylase having 81% or greater sequence identity to SEQ ID NO:1 fermenting is carried out at a temperature in the range of 31°C to 35°C for most or all of a fermentation period.

In some embodiments using the engineered yeast with glucoamylase having 81% or greater sequence identity to SEQ ID NO:1, at the end of the fermentation period the glucose concentration in the fermentation medium of not greater than 1.0 g/L.

In a non-claimed embodiment, the engineered yeast comprises an exogenous nucleic acid encoding a glucoamylase comprising a sequence having 97% or greater, or 98% or greater sequence identity to SEQ ID NO:4 (*Rhizopus delemar* glucoamylase). In an embodiment, the engineered yeast is capable of producing ethanol at a rate of 1.0, 1.5, 2.0, 2.5, 3.0, 3.5, 4.0, 4.5, or 5.0 g/L*h or greater during a fermentation process. In another embodiment, the engineered yeast is capable of producing (a) at least 70g/kg of ethanol in a fermentation medium made from a glucose polymer-containing feedstock having (i) a DE of about 30. In embodiments, the amount of 70g/kg of ethanol may be produced within 48 hours of inoculation in a fermentation medium with the feedstock having. In embodiments, the glucose concentration may not be greater than 5% (wt) in the fermentation medium at the beginning (inoculation) of the fermentation process. In embodiments, the feedstock may provide an amount of glucose polymer-containing feedstock sufficient to produce 70g/kg ethanol, for example about 20 wt% glucose-polymer feedstock in the medium. In embodiments, the feedstock may have one or more of the following properties: the feedstock is a starch hydrolysate; the glucose-polymer has predominantly α-1,4 linkages; the feedstock substantially excludes cellulosic materials (e.g., less than 20%, 15%, or 10% of cellulosic material).

In a related non-claimed embodiment, the invention provides a fermentation method for producing a bioproduct. The method comprises forming a fermentation medium from a glucose polymer-containing feedstock, and then fermenting the fermentation medium using an engineered yeast comprising an exogenous nucleic acid encoding a glucoamylase comprising a sequence having 97% or greater sequence identity to SEQ ID NO:4. Fermentation is carried out over a period to produces the bioproduct, such as ethanol.

In more specific embodiments of any of the preceding fermentation, ethanol is produced during the fermentation period to an amount of 70 g/kg or greater, 90 g/kg or greater, 120 g/kg or greater, 130 g/kg or greater, or 140 g/kg or greater, in the fermentation medium. In some embodiments, in the fermentation method, the glucose polymer-containing feedstock has a dextrose equivalent (DE) that is about 30. In some embodiments, glucose is not greater than 5 wt % of solids materials in the feedstock. Optionally, supplemental glucoamylase can be introduced into the fermentation methods to increase bioproduct titers.

In some non-claimed embodiments using the engineered yeast with glucoamylase having 97% or greater or 98% or greater sequence identity to SEQ ID NO:4, fermenting is carried out at a temperature in the range of 31°C to 35°C for most or all of a fermentation period.

In some non-claimed embodiments using the engineered yeast with glucoamylase having 97% or greater sequence identity to SEQ ID NO:4, at the end of the fermentation period the glucose concentration in the fermentation medium is not greater than 2.0L, or not greater than 1.0 g/L.

In more specific embodiments of any of the preceding yeast embodiments, the engineered yeast is capable of producing 90 g/kg or greater, 120 g/kg or greater, 130 g/kg or greater, or 140 g/kg or greater of ethanol in a fermentation medium made from a glucose polymer-containing feedstock having (i) a DE of about 30.

### BRIEF DESCRIPTION OF THE FIGURE

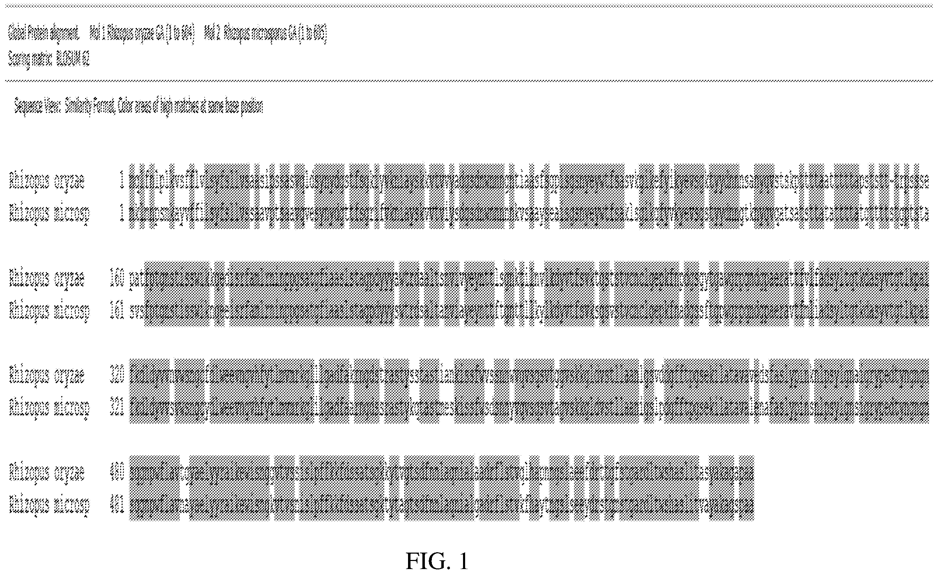

Figure 1 shows an alignment of SEQ ID NO:1 to *Rhizopus oryzae* GA, showing the signal sequence (1-25) and starching binding domain (26-131).

### DETAILED DESCRIPTION

The aspects of the present invention described below are not intended to be exhaustive or to limit the invention to the precise forms disclosed in the following detailed description. Rather a purpose of the aspects chosen and described is so that the appreciation and understanding by others skilled in the art of the principles and practices of the present invention can be facilitated. The scope of protection is determined by the claims.

The claimed invention relates to engineered yeast that expresses a glucoamylase comprising a sequence having 81%, 82%, 83%, 84%, 85%, 90%, 95%, 96%, 97%, 98%, or 99% or greater sequence identity to SEQ ID NO:1 (*Rhizopus microsporus* glucoamylase (GA)), wherein the yeast cell is capable of producing ethanol at a rate of 1g/L*h or greater during a fermentation process and wherein there are 2-8 copies of the exogenous nucleic acid in the cell. Anon-claimed aspect of the invention relates to engineered yeast that expresses a glucoamylase comprising a sequence having 97%, 98%, or 99% or greater sequence identity to SEQ ID NO:4 (*Rhizopus delemar* glucoamylase (GA)). Engineered yeast of the disclosure are able to express and provide glucoamylase enzyme in the culture medium, and the glucoamylases are enzymatically active on glucose polymer substrates such as starch from various plant sources. The glucoamylase activity within the medium can generate mono and disaccharide sugars which can be consumed by the yeast and can be used as a carbon source for the production of a target compound, such as ethanol.

In embodiments of the disclosure, a fermentation medium can be prepared from a feedstock having glucose polymer and minimal glucose. For example, a fermentation medium can be prepared from a starch-containing feedstock having a DE (dextrose equivalent) of 70 or less, 60 or less, 50 or less, 40 or less, 30 or less, 20 or less, 15 or less, 10 or less, or 5 or less. Dextrose equivalent (DE) is a measure of the amount of reducing sugars present in a material (e.g., a sugar product or a starch-containing feedstock), relative to dextrose (a.k.a. glucose), expressed as a percentage on a dry basis. For example, a maltodextrin with a DE of 10 would have 10% of the reducing power of dextrose (which has a DE of 100). A fermentation medium can be prepared from a starch-containing feedstock that does not have a glucose amount that is greater than about 5% wt., or greater than about 4% wt., or greater than about 3% wt., or greater than about 2% wt., or greater than about 1% wt., per total solids in the feedstock. In some fermentation methods the low glucose-containing starch feed stock is added periodically or continuously throughout the fermentation period. The glucoamylases produced by the engineered yeast can be enzymatically active against the starch in the medium and generate glucose which can be used by the yeast for growth and generation of bioproduct. Optionally, the fermentation method can include supplementing the medium with purified glucoamylase, such as glucoamylase obtained from a commercial source, which can further drive enzymatic hydrolysis of the starch and increase growth and titers of bioproduct.

For example, without any commercial glucoamylase enzyme supplementation and using a low glucose feedstock, the engineered yeast of the disclosure can generate an amount of ethanol of about 70 g/kg or greater, such as an amount in the range of about 70 g/kg to about 115 g/kg, about 75 g/kg to about 115 g/kg, about 80 g/kg to about 115 g/kg, about 85 g/kg to about 115 g/kg, about 90 g/kg to about 115 g/kg, about 95 g/kg to about 115 g/kg, about 100 g/kg to about 115 g/kg, or about 105 g/kg to about 115 g/kg of ethanol in the fermentation medium. In embodiments wherein supplemental glucoamylase is added to the medium, greater amounts of ethanol can be produced, such as an amount of 110 g/kg or greater, or 125 g/kg or greater, or 140 g/kg or greater, in the fermentation medium.

In further embodiments, yeast engineered with glucoamylases of the disclosure can exhibit excellent fermentation performance at temperatures greater than standardly used in fermentations (i.e., fermentations at 30°C using *Saccharomyces cerevisiae* as a host organism). For example, in some embodiments using the engineered yeast of the disclosure, fermenting is carried out at a temperature in the range of 31°C to 35°C, or 32°C to 34°C, for most or all of a fermentation period. Even at the higher temperatures, the engineered yeast are able to generate glucoamylase activity in the medium, and promote excellent cell growth and bioproduct production.

In further embodiments, following a period of fermentation, yeast engineered with glucoamylases of the disclosure can provide a desirable final fermentation medium with high levels of bioproduct and low levels of byproduct. In particular, the final fermentation medium can have high levels of ethanol (e.g., 70 g/kg or greater), under stressful fermentation conditions, such as where low levels of glucose, such as 1.0 g/kg, may be present.

The term "exogenous" as used herein, means that a molecule, such as a nucleic acid, or an activity, such as an enzyme activity, is introduced into the host organism. An exogenous nucleic acid can be introduced into the host organism by well-known techniques and can be maintained external to the hosts chromosomal material (e.g., maintained on a non-integrating vector), or can be integrated into the host's chromosome, such as by a recombination event. An exogenous nucleic acid can encode an enzyme, or portion thereof, that is either homologous or heterologous to the host organism.

The term "heterologous" refers to a molecule or activity that is from a source that is different than the referenced molecule or organism. Accordingly, a gene or protein that is heterologous to a referenced organism is a gene or protein not found in that organism. For example, a specific glucoamylase gene found in a first fungal species and exogenously introduced into a second fugal species that is the host organism is "heterologous" to the second fungal organism.

The following SEQ ID NOs are associated with the fungal GA amino acid or protein sequences: SEQ ID NO: 1: *Rhizopus microsporus* GA amino acid sequence; SEQ ID NO:2: *Rhizopus microsporus* GA nucleic acid sequence #1; SEQ ID NO:3: *Rhizopus microsporus* GA nucleic acid sequence #2; SEQ ID NO:4: *Rhizopus delemar* GA amino acid sequence; SEQ ID NO:5: *Rhizopus delemar* GA nucleic acid sequence #1; and SEQ ID NO:6: *Rhizopus delemar* GA nucleic acid sequence #2.

Table 1 is a table of sequence identity (global protein alignment) between *the Rhizopus microsporus* GA amino acid sequence and GA sequence of other known GAs. The reference molecule is *Rhizopus microsporus* GA, and scoring matrix was BLOSUM 62.

**Table 1**

| GA sequence | Start | End | # Match | # Nonmatch | % Match |
|---|---|---|---|---|---|
| *Rhizopus microsporus* | 1 | 605 | - | - | 100 |
| *Rhizopus delemar* | 1 | 604 | 488 | 117 | 80 |
| *Rhizopus oryzae* | 1 | 604 | 485 | 120 | 80 |
| *Mucor ambiguus* | 1 | 609 | 416 | 194 | 68 |
| *Mucor circenello* | 1 | 609 | 415 | 198 | 67 |
| *Choanephora cucurbitarum* | 1 | 622 | 402 | 222 | 64 |
| *Phycomyces blakesleeanus* | 1 | 598 | 369 | 243 | 60 |
| *Arthrobotrys oligospora* | 1 | 621 | 204 | 441 | 31 |

*Rhizopus microsporus* GA and *Rhizopus delemar* GA are members of the glucoamylase enzyme family (E.C. 3.2.1.3) and are amylolytic enzymes that hydrolyze 1,4-linked a-D-glucosyl residues successively from the nonreducing end of oligo- and polysaccharide chains with the release of D-glucose. Alternative names for glucoamylases include amyloglucosidase; γ-amylase; lysosomal α-glucosidase; acid maltase; exo-1,4-α-glucosidase; glucose amylase; γ-1,4-glucan glucohydrolase; acid maltase; 1,4-α-D-glucan glucohydrolase.

Glucoamylases such as *Rhizopus microsporus* GA and *Rhizopus delemar* GA can also cleave α-1,6 bonds on amylopectin branching points. As used herein, the term "amylolytic activity" pertains to these enzymatic mechanisms.

Engineered yeast of the disclosure can include variant(s) of the natural sequences of the *Rhizopus microsporus* GA and *Rhizopus delemar* GA glucoamylase polypeptide and can include one or more amino acid variations, providing for a non-natural polypeptide. Polypeptides of the disclosure can be a portion of the naturally occurring *Rhizopus microsporus* GA and *Rhizopus delemar* GA sequence (such as polypeptides that are truncated at its N-terminus, its C-terminus, or both), while the glucoamylase polypeptide retains amylolytic activity.

N-terminal truncations can be produced by altering the position of the ATG start codon, while ensuring the sequence downstream remains in frame. C-terminal variants can be produced by inserting an in-frame premature stop codon. Random methods such as error-prone PCR could also be employed, and could be combined with growth on starch to ensure peptide function.

Variations in the *Rhizopus microsporus* GA (SEQ ID NO:1) and *Rhizopus delemar* GA (SEQ ID NO:4) sequences can be made with information about the primary sequence of these enzymes, through sequence alignments, and in view of information regarding other glucoamylase enzymes as known in the art. Most glucoamylases, including *Rhizopus microsporus* GA and *Rhizopus delemar* GA, are multidomain enzymes. Many glucoamylases, including *Rhizopus microsporus* GA and *Rhizopus delemar* GA, include a starch-binding domain connected to a catalytic domain via an O-glycosylated linker region, based on known crystal structures from similar enzymes.

Glucoamylases may also have a catalytic domain having a configuration of a configured twisted (alpha/alpha)(6)-barrel with a central funnel-shaped active site. Glucoamylases may have a structurally conserved catalytic domain of approximately 450 residues. In some glucoamylases the catalytic domain generally followed by a linker region consisting of between 30 and 80 residues that are connected to a starch binding domain of approximately 100 residues.

Glucoamylase properties may be correlated with their structural features. A structure-based multisequence alignment was constructed using information from catalytic and starch-binding domain models (see, e.g., Coutinho, P. M., and Reilly, P. J., 1994. Protein Eng. 7:393-400 and 749-760). It has been shown that the catalytic and starch binding domains are functionally independent based on structure-function relationship studies, and there are structural similarities in microbial glucoamylases. From other studies, specific glucoamylase residues have been shown to be involved in directing protein conformational changes, substrate binding, thermal stability, and catalytic activity (see, for example, Sierks, M. R., et al. 1993. Protein Eng. 6:75-79; and Sierks, M. R., and Svensson, B. 1993. Biochemistry 32:1113-1117).

Therefore, the correlation between glucoamylase sequence and protein function is understood in the art, and one of skill could design and express variants of amylolytically active glucoamylases having one or more amino acid deletion(s), substitution(s), and/or additions. For example, in some aspects, the glucoamylase portion of the *Rhizopus microsporus* GA and *Rhizopus delemar* GA can contain a truncated version of a naturally occurring glucoamylase, the truncated version having, in the least, a catalytic and optionally a starch-binding domain having amylolytic activity as described herein.

Truncated forms of glucoamylase have been generated and have been shown to have enzymatic activity. For example, Evans et al. (Gene, 91:131; 1990) generated a series of truncated forms of glucoamylase to investigate how much of the O-glycosylated region was necessary for the activity or stability of GAII, a fully active form of the enzyme lacking the raw starch-binding domain. It was found that a significant portion of the C-terminus could be deleted from GAII with insignificant effect on activity, thermal stability, or secretion of the enzyme.

Lin et.al (BMC Biochemistry 8:9, 2007) teaches there was no loss of glucoamylase activity the starch binding domain located between positions 26-131 in the *Rhizopus oryzae* glucoamylase was deleted. Also, Mertens & Skory (Enz. Microbiol. Technology 40: 874-880, 2007) isolated a natural glucoamylase which lacked a starch binding domain.

Various amino acids substitutions associated with causing a change in glucoamylase activity are also known in the art. Substitution(s) of amino acid(s) at various locations in the glucoamylase sequence have been shown to affect properties such as thermo stability, starch hydrolysis activity, substrate usage, and protease resistance. As such, the current disclosure contemplates use of a *Rhizopus microsporus* GA and *Rhizopus delemar* GA sequence that includes one or more amino acids substitution(s) in the glucoamylase portion of the polypeptide, wherein the substitutions differ from the wild type sequence of the glucoamylase.

For example, U.S. Patent No. 8,809,023 describes a method for reducing the ratio between isomaltose synthesis and starch hydrolysis activity (IS/SH ratio) during the hydrolysis of starch. In particular, a *Trichoderma reesei* glucoamylase (*Tr* GA) is described (total length of 632 amino acids having an N-terminal having a signal peptide) that is modified at with amino acid positions as follows: D44R and A539R; or D44R, N61I, and A539R. This glucoamylase variant is reported to exhibit a reduced IS/SH ratio compared to said parent glucoamylase during the hydrolysis of starch.

As another example, U.S. Patent No. 8,592,194 describes glucoamylase variants with increased thermo stability compared to wild type glucoamylase variants. Also described in this disclosure is the *Trichoderma reesei* glucoamylase but instead one or more amino acid substitutions to the native Tr GA sequence at positions 10, 14, 15, 23, 42, 45, 46, 59, 60, 61, 67, 68, 72, 73, 97, 98, 99, 102, 108, 110, 113, 114, 122, 124, 125, 133, 140, 144, 145, 147, 152, 153, 164, 175, 182, 204, 205, 214, 216, 219, 228, 229, 230, 231, 236, 239, 240, 241, 242, 244, 263, 264, 265, 268, 269, 276, 284, 291, 300, 301, 303, 310, 311, 313, 316, 338, 342, 344, 346, 349, 359, 361, 364, 379, 382, 390, 391, 393, 394, 408, 410, 415, 417, and 418. As an example, the current disclosure contemplates creating variants at amino acid locations in SEQ ID NO:1 and SEQ ID NO:4 corresponding to the respective described positions in the TrGA sequence in order to provide variants with increased thermostability.

The determination of "corresponding" amino acids from two or more glucoamylases can be determined by alignments of all or portions of their amino acid sequences. Sequence alignment and generation of sequence identity include *global alignments* and *local alignments,* which typically use computational approaches. In order to provide global alignment, global optimization forcing sequence alignment spanning the entire length of all query sequences is used. By comparison, in local alignment, shorter regions of similarity within long sequences are identified.

As used herein, an "equivalent position" means a position that is common to the two sequences (e.g., a template GA sequence and a GA sequence having the desired substitution(s)) that is based on an alignment of the amino acid sequences of one glucoamylase or as alignment of the three-dimensional structures. Thus, either sequence alignment or structural alignment, or both, may be used to determine equivalence.

In some modes of practice, the BLAST algorithm is used to compare and determine sequence similarity or identity. In addition, the presence or significance of gaps in the sequence which can be assigned a weight or score can be determined. These algorithms can also be used for determining nucleotide sequence similarity or identity. Parameters to determine relatedness are computed based on art known methods for calculating statistical similarity and the significance of the match determined. Gene products that are related are expected to have a high similarity, such as greater than 50% sequence identity. Exemplary parameters for determining relatedness of two or more sequences using the BLAST algorithm can be as follows.

Inspection of nucleic acid or amino acid sequences for two nucleic acids or two polypeptides will reveal sequence identity and similarities between the compared sequences. Sequence alignment and generation of sequence identity include global alignments and local alignments which are carried out using computational approaches. An alignment can be performed using BLAST (National Center for Biological Information (NCBI) Basic Local Alignment Search Tool) version 2.2.31 software with default parameters. Amino acid % sequence identity between amino acid sequences can be determined using standard protein BLAST with the following default parameters: Max target sequences: 100; Short queries: Automatically adjust parameters for short input sequences; Expect threshold: 10; Word size: 6; Max matches in a query range: 0; Matrix: BLOSUM62; Gap Costs: (Existence: 11, Extension: 1); Compositional adjustments: Conditional compositional score matrix adjustment; Filter: none selected; Mask: none selected. Nucleic acid % sequence identity between nucleic acid sequences can be determined using standard nucleotide BLAST with the following default parameters: Max target sequences: 100; Short queries: Automatically adjust parameters for short input sequences; Expect threshold: 10; Word size: 28; Max matches in a query range: 0; Match/Mismatch Scores: 1, -2; Gap costs: Linear; Filter: Low complexity regions; Mask: Mask for lookup table only. A sequence having an identity score of XX% (for example, 80%) with regard to a reference sequence using the NCBI BLAST version 2.2.31 algorithm with default parameters is considered to be at least XX% identical or, equivalently, have XX% sequence identity to the reference sequence.

A global alignment can align sequences with significant identity to, for example, the SEQ ID NO:1 (*Rhizopus microsporus* GA) or SEQ ID NO:4 *Rhizopus delemar* GA glucoamylase in order to determine which corresponding amino acid position(s) in the target sequence (e.g., a glucoamylase ortholog) can be substituted with the one or more of the amino acid if a glucoamylase variant is used.

In some cases, the substitution can be a conservative substitution, such as where one amino acid of a particular type (e.g., polar, non-polar/aliphatic, positively charged/basic, negatively charged/acidic) is replaced with an amino acid of the same type. Exemplary conservative amino acid substitutions of the present disclosure can involve exchange of one aliphatic or hydrophobic amino acid Ala, Val, Leu, or Ile for another; exchange of one hydroxyl amino acid Ser or Thr for the other; exchange of one acidic amino acid Asp or Glu for the other; exchange of one amide amino acid Asn or Gln for the other, exchange of one basic amino acid Lys, Arg, for His for another; exchange of one aromatic amino acid Phe, Tyr,or Trp, for another, and exchange of one small amino acids Ala, Ser, Thr, Met, or Gly for another.

In embodiments of the disclosure, SEQ ID NO:1 has one or more amino acid mutations which causes it to be less than 100% identical to SEQ ID NO:1. For example, the glucoamylase may have multiple amino acid deletion(s), substitution(s), and/or additions causing it to have about 81% or greater identity to SEQ ID NO:1, 82% or greater, 83% or greater, 84% or greater, 85% or greater, 86% or greater, 87% or greater, 88% or greater, 89% or greater, 90% or greater, 91% or greater, 92% or greater, 93% or greater, 94% or greater, 95% or greater, 96% or greater, 97% or greater, 98% or greater, or 99% or greater identity to SEQ ID NO:1. A variant with a single amino acid substitution has 99.8% identity to SEQ ID NO:1.

In non-claimed embodiments, more than one location in SEQ ID NO:1 can be changed to provide a variant that has to SEQ ID NO:1 that is less than 80%. For example, changes to the signal sequence and deletion of the starch binding domain can provide a variant with less than 80% identity to SEQ ID NO:1, such as about 75%-80% identity to SEQ ID NO:1.

Figure 1 shows an alignment of SEQ ID NO:1 to *Rhizopus oryzae* GA, showing the signal sequence (1-25) and starching binding domain (26-131).

Table 2 is a table of sequence identity (BLAST alignment) of a "core" sequence of SEQ ID NO:1 (lacking signal sequence and starch binding domain) GA sequences of other known GAs. Accession CEG69155.1 is the same sequence as SEQ ID NO:1.

**Table 2**

| Source | Accession # | Start | End | Match | NonMatch | %Match |
|---|---|---|---|---|---|---|
| *Rhizopus microsporus* | CEG69155.1 | 132 | 605 | | | |
| *Rhizopus microsporus* | ORE14155.1 | 132 | 605 | 458 | 16 | 96 |
| *Rhizopus delemar* | EIE75378.1 | 132 | 604 | 397 | 77 | 83 |
| *Rhizopus oryzae* | P07683.2 | 132 | 604 | 394 | 80 | 83 |
| *Rhizopus oryzae* | BAH09876.1 | 132 | 604 | 395 | 79 | 83 |
| *Rhizopus oryzae* | ABB77799.1 | 132 | 604 | 396 | 78 | 83 |

In non-claimed embodiments of the disclosure, SEQ ID NO:4 has one or more amino acid mutations which causes it to be less than 100% identical to SEQ ID NO:4. For example, the glucoamylase may have multiple amino acid deletion(s), substitution(s), and/or additions causing it to have about 98% or greater, or 99% or greater identity to SEQ ID NO:4. A variant with a single amino acid substitution has 99.8% identity to SEQ ID NO:4.

In exemplary embodiments, one or more of locations in SEQ ID NO:1 or SEQ ID NO:4 can be changed to provide a variant. SEQ ID NO:1 and SEQ ID NO:4 also generally include a native "signal sequence." Various other terms may be used to indicate a "signal sequence" as known in the art, such as where the word "signal" is replaced with "secretion" or "targeting" or "localization" or "transit" or leader," and the word "sequence" is replaced with "peptide" or "signal." Generally, a signal sequence is a short amino acid stretch (typically in the range of 5-30 amino acids in length) that is located at the amino terminus of a newly synthesized protein. Most signal peptides include a basic N-terminal region (n-region), a central hydrophobic region (h-region) and a polar C-terminal region (c-region) (e.g., see von Heijne, G. (1986) Nucleic Acids Res. 14, 4683-4690).

In SEQ ID NO:1 and SEQ ID NO:4 the predicted signal sequence is from amino acid 1 to 25 of SEQ ID NO:1 and from amino acid 1 to 25 of SEQ ID NO:4, respectively. A signal sequence can target the protein to a certain part of the cell, or can target the protein for secretion from the cell. For example, it has been shown that the native N-terminal signal sequence of the *S. diastaticus* Glucoamylase STAI gene can target it to the endoplasmic reticulum of the secretory apparatus (for example, see Yamashita, I. et al., (1985) J. Bacteriol. 161, 567-573).

Glucoamylase enzymes of the disclosure can use the native signal sequences of SEQ ID NO:1 and SEQ ID NO:4, or variants thereof, or can be modified to include a heterologous signal sequences. In one aspect, the current invention provides the partial or full replacement of the native signal sequence of SEQ ID NO:1 and SEQ ID NO:4 with a secretion signal based on a sequence at the N-terminal portion of *An aa, Sc* FAKS, Sc AKS, *Sc* MFα1, *Sc* IV, *Gg* LZ, and *Hs* SA as described in U.S. Provisional Patent Application 62/371,681 (published as WO2018027131) and PCT Application No. PCT/US2016/016822 (published as WO2016127083

These secretion signals can be used as a replacement to the native secretion signal of the SEQ ID NO:1 and SEQ ID NO:4, or can be used in addition to the native secretion signal. In view of the addition of the heterologous secretion signal, the proteins may be referred to as "fusion proteins," and annotated as follows: [*An aa-*SS]-[*Rm* GA], [*Sc* IV-SS]-[*Rd* GA], etc.

Possible heterologous N-terminal replacement sequences for the N-terminal of SEQ ID NO:1 and SEQ ID NO:4 include the following. *Sc*-FAKS is a sequence of 90 amino acids derived from the N-terminal portion of the *Saccharomyces cerevisiae* peptide mating pheromone α-factor (e.g., see Brake, A., et al., Proc. Natl. Acad. Sci., 81:4642-4646, 1984; Kurjan, J. & Herskowitz, I., Cell 30:933-943, 1982). *Sc*-MFα1 is amino acids 20-89 Sc-FAKS. *Sc* IV a 19 amino acid N-terminal signal peptide of a sucrose hydrolase enzyme (e.g, see, Carlson M., et al. (1983) Mol. Cell. Biol. 3:439-447). *Gg* LZ (also known as egg white lysozyme) is an 18 amino acid N-terminal signal peptide of a glycoside hydrolase enzyme (e.g, see, Jigami et al. (1986) Gene 43:273-279). *Hs* SA is an 18 amino acid N-terminal signal peptide of a serum (e.g, see, Kober et al. (2013) Biotechnology and Bioengineering; 110:1164-1173.). *Sc* MFα2 is derived from the N-terminus the *Saccharomyces cerevisiae* mating factor alpha 2 gene (*Sc* MFα2). *Sc* PHO5 is derived from the N-terminus of the *Saccharomyces cerevisiae* repressible acid phosphatase (Meyhack et al., EMBO J. 6:675-680, 1982).

Molecular techniques can be performed to create a nucleic acid sequence that is a template for the expression of genes encoding SEQ ID NO:1 or SEQ ID NO:4, or variants thereof. As a general matter, a nucleic acid is prepared to encode a protein comprising SEQ ID NO:1 or SEQ ID NO:4, or variants thereof.

In other aspects, the SEQ ID NO:1 or SEQ ID NO:4, or variants thereof optionally comprises additional sequence that is not present in the native glucoamylase polypeptide. The additional sequence, in some aspects, can provide functionality to the glucoamylase that is not present in the native polypeptide. Additional functionalities include, for example, protease sites or binding sites for other proteins or materials, or linker regions.

Nucleic acids sequences encoding SEQ ID NO:1 or SEQ ID NO:4, or variants thereof, as well as any regulatory sequence (e.g., terminator, promoter, etc.) and vector sequence (e.g., including a selection marker, integration marker, replication sequence, etc.) can, in some modes of practice, be prepared using known molecular techniques. General guidance for methods for preparing DNA constructs (e.g., for the DNA constructs including nucleic acids encoding SEQ ID NO:1 or SEQ ID NO:4 or a variant thereof) can be found in Sambrook et al Molecular Cloning, A Laboratory Manual, Cold Spring Harbor Laboratory Press, Cold Spring Harbor, N.Y., 1989; and Ausubel et al. Current Protocols in Molecular Biology, Greene Publishing and Wiley-Interscience, New York, N.Y., 1993. PCR techniques can be used for modifying nucleic acids encoding SEQ ID NO:1 or SEQ ID NO:4, to optionally introduce one or more mutations in the sequence to provide a variant.

PCR techniques are described in, for example, Higuchi, (1990) in PCR Protocols, pp. 177-183, Academic Press; Ito et al (1991) Gene 102:67-70; Bernhard et al (1994) Bioconjugate Chem. 5:126-132; and Vallette et al (1989) Nuc. Acids Res. 17:723-733. The techniques may optionally include site-directed (or oligonucleotide-mediated) mutagenesis, PCR mutagenesis, and cassette mutagenesis of an earlier prepared DNA encoding a glucoamylase polypeptide.

Alternatively, nucleic acid molecules can be generated by custom gene synthesis providers such as ATUM (Menlo Park, CA) or GeneArt (Life Technologies, Thermo Fisher Scientific).

An expression vector can be constructed to include the glucoamylase nucleic acid sequence operably linked to expression control sequences functional in the host organism. Expression vectors applicable for use in the host organisms include, for example, plasmids, episomes and artificial chromosomes. The vectors can include selection sequences or markers operable for stable integration into a host chromosome. Additionally, the vectors can include one or more selectable marker genes and appropriate expression control sequences. Selectable marker genes also can be included that, for example, provide resistance to antibiotics or toxins, complement auxotrophic deficiencies, or supply critical nutrients not in the culture medium. Expression control sequences can include constitutive and inducible promoters, transcription enhancers, transcription terminators, and the like which are well known in the art.

In some aspects, the nucleic acid can be codon optimized. The nucleic acid template can be the native DNA sequence that codes for the glucoamylase, or the template can be a codon-optimized version that is optimized for expression in a desired host cell. Databases that provide information on desired codon uses in particular host organisms are known in the art.

The DNA construct comprising the glucoamylase nucleic acid can be operably linked to a promoter sequence, wherein the promoter sequence is functional in a host cell of choice. In some aspects, the promoter shows transcriptional activity in a fungal host cell and may be derived from genes encoding proteins either homologous or heterologous to the host cell. In some aspects, the promoter is useful for expression in *S. cerevisiae.* Examples of well-known constitutive promoters include, but are not limited to the cytochrome c promoter (pCYC), translational elongation factor promoter (pTEF), the glyceraldehyde-3-phosphate dehydrogenase promoter (pGPD/TDH3), the phosphoglycerate kinase promoter (PGK), and the alcohol dehydrogenase promoter (pADH). Optionally, an additional factor that controls expression such as an enhancer or the like may also be included on the vector.

The expression vector including the glucoamylase gene can also include any termination sequence functional in the host cell. For example, the termination sequence and the promoter sequence can be from the same cell, or the termination sequence is homologous to the host cell. The termination sequence can correspond to any promoter that is used.

The DNA construct may be introduced into a host cell using a vector. The vector may be any vector which when introduced into a host cell is stably introduced. In some aspects, the vector is integrated into the host cell genome and is replicated. Vectors include cloning vectors, expression vectors, shuttle vectors, plasmids, phage particles, cassettes and the like. In some aspects, the vector is an expression vector that comprises regulatory sequences operably linked to the glucoamylase coding sequence. SEQ ID NOs as described herein can be assembled in the cell by the transformation of multiple smaller DNA fragments (e.g., "SEQ ID NO sub-fragments") with overlapping homology that in total constitute a particular SEQ ID NO. For example, the integration of a desired SEQ ID NO, or portion thereof, at a gene locus in the cell can be accomplished by the co-transformation of two to five DNA sub-fragments, which are subjected to recombination with each other and integration into a genetic locus in the cell having homology to portions of the sub-fragments.

The DNA construct comprising the glucoamylase gene can further include a selectable marker, thereby facilitating the selection in a host cell. For example, the selectable marker can be for transformed yeast. Examples of yeast selectable marker include markers commonly used for selecting for transformed yeast cells. Auxotrophic markers can be used using a gene that controls an auxotrophy, meaning that the gene enables yeast to produce a nutrient required for the growth of the yeast. Examples genes that control auxotrophies include leucine auxotrophy (LEU2), histidine auxotrophy (HIS3), uracil auxotrophy (URA3, URA5), and tryptophan auxotrophy (TRP1).

The DNA construct may be one which is integrated into the genome and replicated together with the chromosome(s) into which it has been integrated. For example, a yeast cell may be transformed with the DNA construct encoding the glucoamylase, and integrating the DNA construct, in one or more copies, in the host chromosome(s). This integration is generally considered to be an advantage, as the DNA sequence is more likely to be stably maintained. Integration of the DNA constructs into the host chromosome may be performed according to conventional methods, such as by homologous or heterologous recombination.

Engineered yeast of the disclosure can include having multiple copies (two or more) of the gene encoding SEQ ID NO:1 or SEQ ID NO:4, or variants thereof. The engineered yeast of the claimed invention has 2-8 copies of the exogenous nucleic acid in the yeast cell. For example, the engineered yeast can be an engineered *Saccharomyces* that has at least first, second, third, and fourth exogenous nucleic acids each including a sequence encoding at least one SEQ ID NO:1, or variants thereof or, in a non-claimed embodiment, SEQ ID NO:4, or variants thereof. If the engineered yeast includes multiple copies of a gene encoding the glucoamylase gene, the nucleic acid sequences of the copies can be the same or different from one another. Exemplary methods and yeast strains that have been engineered to include multiple copies of glucoamylase genes are described in International Application serial no. PCT/US16/24249, filed March 25, 2016 (Miller, et al).

The engineered yeast can also optionally include introduction of exogenous nucleic acid sequences, changes to regulatory elements that either upregulate or down regulate expression of genes; increase in gene copy numbers, and deletions or mutations that eliminate expression, reduce expression, or increase expression or activity of a gene or gene product. The heterologous modification can include one or more of the following: the use of a promoter that is different than the native promoter of the desired gene; the use of a terminator that is different than the native terminator of the desired gene; the introduction of the gene at a location in the genome that is different than its native location; the introduction of multiple copies of the desired gene.

An additional genetic modification that can be included in the engineered yeast is the alteration or introduction of an enzyme activity that converts a low molecular weight non-glucose sugar to glucose. For example, one optional additional genetic modification affects or introduces isomaltase activity in the engineered yeast during growth on glucose. Isomaltase can convert isomaltose to glucose by hydrolyzing the 1,6 ether linkage in isomaltose. An isomaltase may also exhibit cross activity for hydrolyzing the 1,4 ether linkages in maltose. The genetic modification can cause isomaltase activity to be introduced into the cell, cause an increased amount of isomaltase in the cell, and/or cause an increase in isomaltase activity.

In some embodiments further to the glucoamylase gene, the engineered cell may include a heterologous isomaltase gene, or an isomaltase gene under the control of a heterologous promoter that provides increased expression in the cell, or present in multiple copies in the cell. For example, an isomaltase (IMA) gene under the control of a heterologous promoter, such as a PDC promoter can be engineered into the yeast.

Examples of isomaltase genes that can be introduced into an engineered yeast include, but are not limited to *Saccharomyces cerevisiae* IMA1 *(*P53051), *Saccharomyces cerevisiae IMA2* (Q08295), *Saccharomyces cerevisiae* IMA3 (P0CW40), *Saccharomyces cerevisiae* IMA4 (P0CW41), *Saccharomyces cerevisiae* IMA5 (P40884), *Bacillus subtilis* malL (O06994)*, Bacillus cereus* malL (P21332), *Bacillus coagulans* malL (Q45101), *Bacillus sp.* malL (P29093), *etc.* Preferably the isomaltase gene encodes for a polypeptide having greater than 80%, 85%, 90%, 95%, 98% or 99% sequence identity with the amino acid sequence of accession number NP 011803.3 (*Saccharomyces cerevisiae* IMA1).

In some embodiments, the engineered yeast can further include a genetic modification that provides a starch-degrading polypeptide that is different than the glucoamylase. For example, the genetic modification can be one that introduces a nucleic acid encoding a different polysaccharide-degrading enzyme, such as an exogenous or modified alpha-amylase, a betaamylase, a pullulanase, or an isoamylase. The genetic modification may also be one that increases the amount of an endogenous or an exogenous starch-degrading polypeptide in the cell, such as by placing the gene under control of a strong promoter, or providing the gene in multiple copies in the cell, such as multiple copies of the gene integrated into the genome, or multiple copies present on a non-chromosomal construct (e.g., a plasmid).

In some embodiments, the engineered yeast can further include a genetic modification that provides an exogenous or modified sugar transporter gene (such as an isomaltose transporter); See, for example, commonly assigned U.S. application Serial Number 62/268,932 filed December 17, 2015, entitled "Sugar Transporter-Modified Yeast Strains and Methods for Bioproduct Production," published as WO2017106739.

Various host cells can be transformed with a nucleic acid encoding SEQ ID NO: 1 or SEQ ID NO:4, or a variant thereof. According to the claimed invention, the host cell is a yeast cell. In non-claimed aspects, the nucleic acid including the glucoamylase gene is present in a bacterial cell. The bacterial cell can be used, for example, for propagation of the nucleic acid sequence or for production of quantities of the polypeptide.

In other non-claimed aspects, the host cell is a eukaryotic cell, such as a fungal cell.

In other aspects, the heterologous glucoamylase can be purified for use in an enzyme composition, either alone or in combination with other enzymes.

In some aspects, the host cell has tolerance to a higher amount of a bioderived product, such as ethanol, in the fermentation medium. In some aspects, the host cell is an "industrial yeast" which refers to any yeasts used conventionally in ethanol fermentation. Examples include sake yeasts, shochu yeasts, wine yeasts, beer yeasts, baker's yeasts, and the like. Sake yeasts demonstrate high ethanol fermentability and high ethanol resistance and genetic stability. Typically, an industrial yeast has high ethanol resistance and preferably is viable at ethanol concentrations of 10% or greater.

In exemplary aspects, the yeast including the glucoamylase gene is a*S*. *cerevisiae* yeast. Some *S. cerevisiae* strains have high tolerance to ethanol. Various strains of ethanol tolerant yeast are commercially available, such as RED STARTM and ETHANOL REDTM yeast (Fermentis/Lesaffre, USA), FALITM (Fleischmann's Yeast, USA), SUPERSTART and THERMOSACCTM yeast (Ethanol Technology, Wis., USA), BIOFERMTM AFT and XR (NABC-North American Bioproducts Corporation, GA, USA), GERT STRAND (Gert Strand AB, Sweden), SUPERSTARTTM (Alltech), ANGELTM (Angel Yeast Ltd, China) and FERMIOLTM (DSM Specialties).

Industrial yeasts are typically prototrophic and therefore do not have an auxotrophic marker suitable for selecting for a transformant. If the yeast does not have the genetic background that would otherwise facilitate retention of the glucoamylase gene of SEQ ID NO:1 or SEQ ID NO:4, or variant thereof, within the cell upon transformation, the host cell can be engineered to introduce one or more genetic mutation(s) to establish use of a marker gene in association with and to maintain the glucoamylase gene in the cell. For example, a commercially available ethanol tolerant yeast cell can be genetically modified prior to introducing the glucoamylase gene in the cell.

A marker for a different auxotrophy can be provided by disrupting the gene that controls the auxotrophy. In one mode of practice, an ethanol tolerant strain of yeast is engineered to disrupt copies of one or more genes that control auxotrophies, such as LEU2, HIS3, URA3, URA5, and TRP1. In the case of providing uracil auxotrophy, for example, a normal ura3 gene of an ethanol tolerant yeast can be replaced with an ura3⁻ fragment obtained from a uracil auxotrophic mutant (for example, a *Saccharomyces cervisiae* MT-8 strain) to disrupt the normal ura3 gene. In the case of a ura3 gene-disrupted strain, the presence/absence of a marker can be easily identified or selected by taking advantage of the fact that a ura3 gene-disrupted strain is able to grow in a medium containing 5-fluoroorotic acid (5-FOA) while a normal ura3 strain (wild-type yeast or usual industrial yeast) is not able to grow. In the case of a lys2 gene-disrupted strain, the presence/absence of a marker can be easily identified or selected by taking advantage of the fact that a lys2 gene-disrupted strain is able to grow in a medium containing α-aminoadipic acid while a normal lys2 strain (wild-type yeast or usual industrial yeast) is not able to grow. Methods for disrupting an auxotrophy-controlling gene and for selectively separating auxotrophy- controlling gene mutants may be used depending on the auxotrophy employed. Alternatively, one can employ dominant selection markers, such as the amdS from *Aspergillus nidulans* (U.S. Patent No. 5,876,988), which allows for growth on acetamide as the sole nitrogen source; or *ARO4-OFP,* which allows for growth in the presence of fluoro-phenylalanine (Fukuda *et. al.*)*.* These markers can be used repeatedly using the recyclable cre-loxP system, or alternatively can be used to create auxotrophic strains that allow additional markers to be utilized.

After the host cell has been engineered to provide a desired genetic background for introduction of the glucoamylase gene, the gene construct is introduced into a cell to allow for expression. Methods for introducing a gene construct into a host cell include transformation, transduction, transfection, co-transfection, electroporation. In particular, yeast transformation can be carried out using the lithium acetate method, the protoplast method, and the like. The gene construct to be introduced may be incorporated into a chromosome in the form of a plasmid, or by insertion into the gene of a host, or through homologous recombination with the gene of a host. The transformed yeast into which the gene construct has been introduced can be selected with a selectable marker (for example, an auxotrophic marker as mentioned above). Further confirmation can be made by measuring the activity of the expressed protein.

The transformation of exogenous nucleic acid sequences including the glucoamylase gene can be confirmed using methods well known in the art. Such methods include, for example, nucleic acid analysis such as Northern blots or polymerase chain reaction (PCR) amplification of mRNA, or immunoblotting for expression of gene products, or other suitable analytical methods to test the expression of an introduced nucleic acid sequence or its corresponding gene product. It is understood by those skilled in the art that the exogenous nucleic acid is expressed in a sufficient amount to produce the desired product, and it is further understood that expression levels can be optimized to obtain sufficient expression using methods well known in the art and as disclosed herein.

The engineered yeast of the disclosure can be provided in any suitable form. In some aspects, the non-natural yeast is dehydrated to form a dry yeast composition. The dry yeast composition can have increased shelf life over wet compositions.

Fermentation using a host cell expressing the glucoamylase gene can be performed in a fermentation medium made from a feedstock derived from a starch and/or sugar containing plant material, referring to a starch and/or sugar containing plant material derivable from any plant and plant part, such as tubers, roots, stems, leaves and seeds. Starch and/or sugar comprising plant material can be obtained from cereal, such as barley, wheat, maize, rye, sorghum, millet, barley, potatoes, cassava, or rice, and any combination thereof. The starch- and/or sugar comprising plant material can be processed, such as by methods such as milling, malting, or partially malting. In some aspects, the starch material can be from corn flour, milled corn endosperm, sorghum flour, soybean flour, wheat flour, biomass derived starch, barley flour, and combinations thereof. Starch-containing feedstocks used to form a fermentation medium can be made from any of these plant materials.

In some aspects, a feedstock used to form a fermentation medium can include a treated starch. For example, the fermentation medium can include a partially hydrolyzed starch. The partially hydrolyzed starch can include high molecular weight dextrins and high molecular weight maltodextrins. Collectively, starch, dextrins, maltodextrins, and any other polymerized form a glucose are glucose polymers. Partially hydrolyzed starches and preparation thereof are well known in the art. Partially hydrolyzed starches can be prepared by heating the starch with an acid such as hydrochloric or sulfuric acid at a high temperature and then neutralizing the hydrolysis mixture with a suitable base such as sodium carbonate. Alternatively, partially hydrolyzed starches can be prepared by an enzymatic process, such as by adding alpha-amylase to a starch preparation. An alpha amylase can cause the endohydrolysis of (1→4)-alpha-D-glucosidic linkages in polysaccharides containing three or more (1→4)-alpha-linked D-glucose units. A partially hydrolyzed starch product can be used that have amounts of starch and starch degradation products within desired ranges. Partially hydrolyzed starch includes preparations having minimal hydrolysis (e.g., a DE of 5, having little dextrose) to preparations having substantial hydrolysis (e.g., a DE of 95, predominantly dextrose).

The feedstock can be a "liquefact", which is corn starch that has undergone liquefaction, with a dextrose equivalents in the range of about 10 to about 15. A corn wet milling process can be used to provide steep-water, which can be used for fermentation. Corn kernels can be steeped and then milled, and separated into their major constituent fractions. Light steep water is a byproduct of the steeping process, and contains a mixture of soluble proteins, amino acids, organic acids, carbohydrates, vitamins, and minerals.

In some aspects, the feedstock can be dry grind corn, i.e., most or all of the corn kernel components are included in the fermentation feedstock. The dry-grind corn process is the most common technology for converting corn to ethanol in the U.S. Some aspects of dry-grind processing differ from the wet milling process (which uses liquefact), including, but not limited to adding urea to provide sufficient nitrogen for fermentation. The primary aspects of dry-grind processes for producing ethanol are well known in the art.

Feedstocks derived from any of the plant materials described herein generally include a "glucose polymer" which refers to those polymers including two or more glucose residues. Shorter glucose polymers including glucose dimers (e.g., maltose), trimers (e.g., triose), and those up to about 10 glucose units, which may also be referred to as "glucose oligomers." Feedstocks of hydrolyzed starch preparations with a DE in the range of about 2 to about 20 include predominantly maltodextrins, which include glucose polymers having a DP of 3 (540 Da) to over about 5000 (10⁶ Da). For example, a starch preparation with a DE 2 includes most maltodextrins in the range of 200,000 to about 1 x 105 Da, and DEs in the range of about 10 to about 20 have most maltodextrins in the range of about 540 to about 100,000 Da. Degree of polymerization (DP) refers to the number of sugar monomer residues in a glucose polymer.

Based on the DE of the partially hydrolyzed starch, the concentrations (% wt) of glucose (DP 1), maltose (DP 2), triose (DP 3), and longer glucose polymers of DP 4+ can be known in the composition. Table 3 provides concentrations (% wt) of various meric forms of glucose at various DE points, as understood in the art.

**Table 3**

| ***DE*** | **Glucose (DP 1)** | **Maltose (DP 2)** | **Triose (DP 3)** | **DP 4-6** | **DP 7+** |
|---|---|---|---|---|---|
| ***5*** | <1 | 1 | 2 | 7 | 90 |
| ***10*** | <1 | 3 | 4 | 15 | 78 |
| ***15*** | <1 | 6 | 7 | 21 | 66 |
| ***20*** | <1 | 8 | 9 | 29 | 53 |
| | | | | DP 4+ | |
| ***28*** | 8 | 8 | 11 | 73 | |
| ***36*** | 14 | 11 | 10 | 65 | |
| ***43*** | 19 | 14 | 12 | 55 | |
| ***53*** | 28 | 18 | 13 | 41 | |
| ***63*** | 36 | 31 | 13 | 20 | |
| ***66*** | 40 | 35 | 8 | 17 | |
| ***95*** | 95 | 3 | 0.5 | 1.5 | |
| ***100*** | 100 | 0 | 0 | 0 | |

Benefits of the engineered yeast of the current disclosure allow use of fermentation mediums made from feedstocks with low DEs, such as feedstocks having a DE of less than about 40, less than about 30, less than about 20, less than about 15, or less than about 10. Such feedstocks may require the need for using exogenous starch-degrading enzymes to generate glucose for cell growth and bioproduct formation. However, starch-containing feedstocks with higher DEs can be used in methods with engineered yeast of the disclosure, and the engineered yeast can still provide fermentation benefits. For example, methods of the invention may use afeedstock including partially hydrolyzed starch having a DE of not greater than about 75, or not greater than about 70, and greater than about 35, or greater than about 40, and more preferably in the range of about 45 to about 65. A DE in the range of about 45 to about 65 means that glucose is present in the feed composition in the range of about 19% to about 40 % (wt), maltose in the range of about 14 to about 35 % (wt), triose in the range of about 8 to about 12 % (wt), and glucose polymers having a DP of 4 or greater in the range of about 17% to about 55%. The percentages are based on the total amount of all "meric" forms of glucose in the composition. Fermentation and addition of starch-containing feedstocks can be carried out to provide glucose and glucose polymer within desired ranges as expressed as a percentage of the total amount of all "meric" forms of glucose in the composition.

In aspects of the disclosure, given production and secretion of the glucoamylase from the engineered yeast into the fermentation medium, the fermentation method may omit addition of purified or enriched commercial glucoamylase into the medium, or at least allow significantly less commercial glucoamylase to be used in a fermentation method. For example, the engineered yeast of the disclosure can allow addition of commercial glucoamylase to be eliminated or at least reduced by about 50%, 60%, 70%, 80%, 90%, or 95%. 100% reduction can be attained using the yeast described herein, especially if a longer fermentation period, for example 60 hours is used. Typically, amounts of glucoamylase in the range of about .014-.071 AGU/g DS would be used in fermentation methods that do not use a glucoamylase-secreting engineered yeast.

The benefits of using yeast engineered to express a glucoamylase enzyme according to SEQ ID NO:1 or SEQ ID NO:4, can be understood by fermenting the yeast in a fermentation medium made from a liquified corn mash having a low DE, such as not greater than about 50, not greater than about 35, not greater than about 30, or not greater than about 25, or not greater than about 20, or not greater than about 15, such as in the range of about 2 to about 20, about 2 to about 15, or about 5 to about 5, and fermenting the medium to generate ethanol. The feedstock used to prepare the fermentation medium may optionally be described in terms of glucose concentration as an overall percentage of fermentable carbohydrates (glucose and glucose polymers) in the feedstock. For example, the fermentation medium can be made from a starch feedstock having a glucose concentration of about 2 % or less, or about 1 % or less, such as in the range of about 0.1% to about 2%, 0.1% to about 1.5%, or to about 0.1% to about 1%. At the end of the fermentation period, the ethanol concentration is 70 g/kg or greater in the fermentation media.

To determine if a yeast expressing a glucoamylase is capable of producing an ethanol concentration of 70 g/kg or great in the fermentation media at the end of a fermentation period, the following test can be conducted: First a fermentation medium using a starch feedstock having a DE of 30 +/- 2 is produced by preparing a corn mash (or liquefied corn mash) using a predetermined amount of yellow dent #2 corn that is milled and passed through a US#20 sieve. Overs (twice-ground corn that was retained on a US#20 sieve) are added back at a X:Y ratio of overs to sieved corn (0.020 overs/total corn mass ratio). The moisture content is measured by the halogen moisture balance method to determine the dry weight of the milled corn. Water is added to create a 32% slurry (w/w, dry weight basis). Concentrated sulfuric acid is added to reach a pH between 5.7-5.9. Calcium chloride dihydrate powder is added to achieve a Calcium concentration of 35ppm. Amylase (Liquozyme^{™} Novozymes Liquozyme Supra 2.2X) is added based on the corn dry starch weight at a dosage ratio of 2.84kg/ton dry basis starch dosage and the slurry is transferred to a Buchi Rotovapor R-220 flask equipped with an oil bath preset at 85°C. The reaction is allowed to proceed for 2 hours, stopping once the dextrose equivalents (DE) reaches 30 +/- 2 by reducing the temperature to between 34-36°C. The pH is adjusted to 5.0 with additional concentrated sulfuric acid. The DE is determined by using an osmometer (Advanced^{™} Model 3D3 and Precion system Model Osmette XL^{™}). Sugar and oligocarbohydrates contents are determined using HPLC with Aminex HPX-87H column (300 mm x 7.8 mm) at 60 C, 0.01N sulfuric acid mobile phase, 0.6 mL/min flow rate. To the starch feedstock a 50% urea solution and 10% ampicillin solution are added, targeting a final concentration of 1900ppm and 5ppm, respectively. A single fermentation typically contains 50g of corn mash, 190ul of 50% urea, and 2.5ul of 10% ampicillin. A typical fermentation vessel is a baffled 250ml shake flask fitted with an air-lock. The air lock should contain four to five milliliters of canola oil. The flask is inoculated to a starting OD₆₀₀ of 0.1, using a cell slurry made by scraping a fresh YPD plate into 1ml of sterile water, Inoculate the fermentation medium with an engineered yeast that is ethanol tolerant (e.g., ETHANOL RED^{®}) having an exogenous nucleic acid that expresses SEQ ID NO:1 or SEQ ID NO:4 to an OD₆₀₀ of 0.1. Fermentation is carried out in flasks at 30°C with shaking in an orbital shaker at 100 rpm for approximately 48 hours. At 48 hours, as sample is analyzed for the concentration of ethanol, and optionally other compounds such as glucose, by high performance liquid chromatography with refractive index detector.

Without any commercial glucoamylase supplementation and using the feedstock described above, typical ethanol titers in the range of about 115 g/kg to about 135 g/kg can be observed using an engineered yeast expressing a glucoamylase of SEQ ID NO:1 or SEQ ID NO:4, or a variant thereof. Greater ethanol titers can be achieved with modifications to the yeast and/or the fermentation conditions. For example, in some embodiments wherein supplemental commercial glucoamylase is added to the medium, greater amounts of ethanol can be produced, such as an amount of 110 g/kg or greater, or 125 g/kg or greater, or 140 g/kg or greater, in the fermentation medium. In addition to the higher final ethanol titers, the fermentation rate can also be increased as free glucose may no longer be limiting the fermentation. Addition of the commercial glucoamylase further acts on starch polymers to create more glucose in the fermentation medium, resulting in increased cell growth and higher ethanol titers.

To achieve an ethanol concentration of 110 g/kg or greater in the fermentation media at the end of a fermentation period, the following test can be conducted. Prepare a starch feedstock and fermentation media as previously described. Supplement the fermentation medium with commercial glucoamylase enzyme (Spirizyme Fuel HS, Novozymes) to provide an additional glucoamylase activity in the medium. 0.097 AGU/g DS, or 30% of the dose required for the wild type can provide a benefit. Glucoamylase activity (AGU) is defined as the amount of enzyme which hydrolyzes 1 micromole maltose per minute under the standard conditions 37°C, using 23.2 mM maltose in 100mM acetate buffer pH 4.3, using a reaction time of 5 minutes. Innoculate the fermentation medium with an engineered yeast that is ethanol tolerant (e.g., ETHANOL RED^{®}) having an exogenous nucleic acid that expresses SEQ ID NO:1 or SEQ ID NO:4. Carry out fermentation for a period of 48 hours at 30°C. With the commercial glucoamylase supplementation and using a low glucose feedstock, typical ethanol titers in the range of about 110 g/kg to about 160 g/kg can be observed.

Test 1 is a method as described in the preceding paragraphs when done at 30°C without commercial GA supplementation, Test 2 is a method as described in the preceding paragraphs when done at 30°C with 0.097 AGU/g DS GA supplementation. Test 3 is a method as described in the preceding paragraphs when done at 33.3°C without commercial GA supplementation. Test 4 is a method as described in the preceding paragraphs when done at 33.3°C with 0.0.097DS GA supplementation. A preferred yeast is one that can produce a minimum of 70g/kg in Test 1 AGU/g (all of the GA strains) and a minimum of 130g/kg in test 4 (the 2X Rmic and 4X Rdel strains).

In further embodiments, following a period of fermentation, yeast engineered with glucoamylases of the disclosure can provide a desirable final fermentation medium with high levels of bioproduct (e.g., ethanol) and low levels of byproduct. For example, the final fermentation medium can have high levels of glucose (e.g., 70 g/kg or greater, 90 g/kg or greater, 110 g/kg or greater, 125 g/kg or greater, or 140 g/kg or greater), and low levels of glucose, such as 1.0 g/kg or less (e.g., 0.9 g/kg or less, 0.8 g/kg or less, 0.7 g/kg or less, 0.6 g/kg or less, or 0.5 g/kg or less). In the final fermentation medium with high ethanol titers, low glucose is beneficial as it improves downstream processes, such as separation of components (e.g., ethanol) in the final fermentation medium.

The fermentation medium includes water and preferably includes nutrients, such as a nitrogen source (such as proteins), vitamins and salts. A buffering agent can also be present in the fermentation medium. Other components may also be present in the fermentation broth after a period of fermentation, such as fermentation products which can accumulate as the fermentation progresses, and other metabolites. Optionally, the fermentation broth can be buffered with a base such as calcium hydroxide or calcium carbonate, ammonia or ammonium hydroxide, sodium hydroxide, or potassium hydroxide in order to maintain a pH at which the organism functions well.

The fermentation medium can optionally include one or more of the following enzymes that are different than the glucoamylase of SEQ ID NO:1 or SEQ ID NO:4, or variant thereof. Exemplary other enzymes include alpha amylases, beta-amylases, peptidases (proteases, proteinases, endopeptidases, exopeptidases), pullulanases, isoamylases, cellulases, hemicellulases, endo-glucanases and related beta-glucan hydrolytic accessory enzymes, xylanases and xylanase accessory enzymes, acetolactate decarboxylases, cyclodextrin glycotransferases, lipases, phytases, laccases, oxidases, esterases, cutinases, granular starch hydrolyzing enzymes and other glucoamylases. These other enzymes can optionally be added to the fermentation medium or the starch-containing feedstock, such as by using a purified commercial preparation of the enzymes. Alternatively, one or more of the other enzymes can be secreted from the engineered yeast expressing SEQ ID NO:1 or SEQ ID NO:4, or from a different engineered cell.

The engineered yeast of the current disclosure can optionally be described in terms of the engineered yeast's specific growth rate. The growth rate of yeast can be defined by L = log(numbers) where numbers is the number of yeast cells formed per unit volume (mL), versus T (time).

The fermentation is carried out under conditions so that fermentation can occur. Although conditions can vary depending on the particular organism and desired fermentation product, typical conditions include a temperature of about 20°C or greater, and more typically in the range of about 30°C or greater. During fermentation the reaction mixture can be mixed or agitated. In some modes of practice, the mixing or agitation can occur by the mechanical action of sparging gas to the fermentation broth. Alternatively, direct mechanical agitation such as by an impellor or by other means can be used during fermentation.

The disclosure also provides non-natural yeast that have the ability to grow, and/or can produce a fermentation product at temperatures that are greater than those in which yeast, such as *Saccharomyces cerevisiae,* typically are used in fermentation processes. For example, *S. cerevisiae* typically have optimal growth at a temperature of about 30°C. However, engineered yeast of the disclosure can grow and provide excellent bioproduct (e.g., ethanol) titers at higher temperatures, and can also provide low residual glucose. For example, in some embodiments using the engineered yeast of the disclosure, fermenting is carried out at a temperature in the range of 31°C to 35°C, or 32°C to 34°C, for most or all of a fermentation period. Even at the higher temperatures, the engineered yeast are able to generate glucoamylase activity in the medium, and promote excellent cell growth and bioproduct production.

During a fermentation process the fermentation medium can reach an elevated temperature such as about 32°C or about 32°C or greater during one or more time(s) during the fermentation process. The temperature can be elevated during part of the fermentation period, or during the entire fermentation period. The temperature can be elevated for 5 minutes of greater, 10 minutes of greater, 30 minutes or greater, 1 hour or greater, 2 hours or greater, 5 hours or greater, or 10 hours or greater. The time of elevated temperature can also be expressed as a total of the overall fermentation period, such as about 0.1% to 100%, about 0.1% to about 75%, about 0.1% to about 50%, about 0.1% to about 25%, about 0.1% to about 10%, about 0.1% to about 5%, about 0.1% to about 2.5%, about 0.1% to about 1%, or about 0.1% to about 0.5% of the fermentation period.

The engineered yeast can also provide a commercially relevant titer of ethanol during or after the period of elevated temperature. For example, during or after the period of elevated temperature, for example, the ethanol titer can be in the range of about 110 g/L to about 170 g/L, in the range of about 125 g/L to about 170 g/L, or in the range of about 140 g/L to about 170 g/L. Accordingly, the engineered yeast described herein can produce ethanol at a commercially useful titer during or after a period of high temperature that would typically cause issues in other currently available yeast strains used in ethanol-producing fermentation processes. Such issues include but are not limited to: death to a significant percentage of yeast cells; deleterious effects on the ability of the yeast to reproduce; and/or reduction or elimination of the ability of the yeast to produce a fermentation product.

Miller et al. (both WO2016127083, filed Feb. 6, 2015, and PCT/US17/045493, filed Aug. 4, 2017) describes the utility of swapping the leader sequence on several glucoamylases, but also highlights the need for additional modifications to the host to achieve acceptable ethanol/glucose titers at the elevated temperatures. Specifically, Miller describes the effect of expressing the Mfalpha2-R. oryzae GA on ethanol and residual glucose titers at two different temperatures, 30°C and 33.3°C. Temperature is a well-known antagonist to healthy ethanol fermentations, and producers spend a significant amount of capital and operating cost in terms of cooling capacity to keep their fermenters in the safe zone, typically less than 34°C. Heterologous protein production is also a well described stressor in engineered organisms, as energy directed towards cell growth and maintenance is diverted to non-natural production processes as described in Mattanovich et. al (Journal of Biotechnology 113, 2004). Alleviating the burden of heterologous protein production has been an area of intense focus over the past several decades, in all aspects of biotechnology (e.g. pharma, industrial enzymes, etc), and is not limited to the yeast *Saccharomyces cerevisiae.* Classical techniques and targeted pathway engineering, two primary methods to overcome the obstacles of producing protein and maintaining healthy host performance have resulted in some success (Payne et. al 2008, Gasser et. al 2007, Valkonen et. al 2003). These results also indicate that there is no one solution to the problem, and a solution for one protein may not work for another.

In some cases, fermentation is carried out in industrial capacity fermenters in order to achieve commercial scale economic benefits and control. In an aspect, the fermentation is carried out in a fermenter that has a capacity of about 10,000 liters or more.

The pH of the fermentation medium can be adjusted to provide optimal conditions for glucoamylase activity, cell growth, and fermentation activity to provide a desired product, such as ethanol. For example, pH of the solution can be adjusted to in the range of 3 to 5.5. In one mode of practice, the pH of the fermentation medium is in the range of 4 to 4.5.

As noted above, the present fermentation process uses genetically modified yeast expressing a sequence having 81% or greater sequence identity to SEQ ID NO:1, or a variant thereof, and capable of secreting the enzyme produced into the fermentation medium. In a non-claimed embodiment the fermentation process uses genetically modified yeast expressing SEQ ID NO:4 or a variant thereof, and capable of secreting the enzyme produced into the fermentation medium. These enzymes are therefore directly exposed to the broth conditions and affect the carbohydrate composition in the fermentation medium. In the fermentation medium the glucoamylase can cause hydrolysis and release of D-glucose from the non-reducing ends of the starch or related oligo- and polysaccharide molecules by cleaving alpha(1,4) and alpha-(1,6) glucosidic bonds.

Starch may also be acted on by one or more other amylases (e.g., alpha-amylase) present in the fermentation medium. For example, if alpha-amylase is present in the fermentation medium it can cause partial hydrolysis of precursor starch and cause a partial breakdown of the starch molecules by hydrolyzing internal alpha-(1,4)-linkages.

In some modes of practice, the fermentation is carried out as a single batch until completion. In other modes of practice, the fermentation is carried out as a fed batch fermentation process. In this mode of practice, a first portion of a total amount of starch material to be fermented is added to the fermentation medium wherein the glucoamylase enzyme acts on the starch to cause formation of glucose to be used as a substrate for fermentation. Additional starch material can be added in one or more portions to provide more substrate for the glucoamylase enzyme in the medium. The addition of starch can be regulated and the formation of glucose can be monitored to provide efficient fermentation.

In some modes of practice, the fermentation is carried out in a continuous mode of operation. In this mode, multiple fermenters operate in series in which a starch hydrolysate is supplied in the first fermenter, which is fed to second fermenter and so on until the starch hydrolysate is converted to ethanol. Continuous operation can be operated using between 2-7 fermenters.

In some modes of practice, a portion of the total amount of starch material is added to the fermentation broth using a variable rate addition system. Examples of such systems include a variable speed pump or a metering valve (such as a throttle valve) operably connected to a pump, which pump or valve can be utilized to vary the amount of starch material introduced into the fermentation broth over time. In some modes of practice, during the addition of a portion of the starch material, glucose concentration is monitored by a real-time monitoring system.

Real-time monitoring systems include systems that directly monitor glucose concentration and systems that indirectly monitor glucose concentration. Examples of real-time monitoring systems that typically directly monitor glucose concentration include systems based on infrared (IR) spectroscopy, near-infrared (NIR) spectroscopy systems, Fourier transform infrared (FTIR) systems, systems based on refractive index, automated enzyme based measurement systems such as a YSI 2950 Biochemistry Analyzer sold by YSI Life Sciences systems, high performance liquid chromatography (HPLC) based systems, gas chromatography (GC) based systems, and other real-time monitoring systems known to one of skill in the art. Additionally real-time monitoring systems that indirectly monitor/measure the glucose concentration of a fermentation process can be developed by determining the typical carbon distribution in a particular fermentation process and correlating the glucose concentration present in the fermentation broth to another parameter exhibited by the fermentation, such as, for example, a correlation of the glucose level present in the fermentation broth with a measurement of the carbon dioxide evolution rate and the amount of carbon dioxide present in an off-gas stream from the fermentation vessel. The carbon dioxide can be readily measured through use of a mass spectrometer or other suitable instrumental technique for measuring the components of the off-gas stream. In a preferred aspect, the glucose concentration is monitored by a real-time monitoring system using infrared spectroscopy. In another aspect, the glucose concentration is monitored by a real-time monitoring system using near-infrared spectroscopy. The real time monitoring systems interface with equipment that controls the introduction of starch material into the fermentation broth to modulate the formation of glucose to a desired concentration in the fermentation broth.

During the fermentation process a sample of the fermentation medium can be taken to determine the amount of glucoamylase activity in the medium. The amount of glucoamylase activity in the medium can be referred to as extracellular glucoamylase activity as it corresponds to glucoamylase secreted from the engineered yeast. In some modes of measuring, the amount of glucoamylase activity in the medium can be determined by the amount of glucoamylase activity per amount of biomass per volume of medium.

Measuring the glucoamylase activity in the fermentation medium can be another way of reflecting the benefits of using yeast engineered to express a glucoamylase enzyme according to SEQ ID NO:1 or SEQ ID NO:4. Such a test can be carried out by using a fermentation medium made from a low DE feedstock, high DE feedstock, or anything in between. During the fermentation process a sample of medium is taken and the biomass amount and the enzyme activity are determined. As used herein "biomass" refers to the weight of the engineered yeast, which can be measured in grams of dried cell weight per liter of medium (DCW/L).

In some modes of practice, the fermentation period is about 30 hours or greater, about 40 hours or greater, about 50 hours or greater, or about 60 hours or greater, such as a period of time in the range of about 40 to about 120 hours, or 50 to about 110 hours.

The fermentation product (also referred to herein as a "bio-derived product" or "bioproduct") can be any product that can be prepared by enzymatic degradation of a starch material by the glucoamylase, formation of glucose, and fermentation of glucose. In some aspects, the fermentation product is selected from the group consisting of: amino acids, organic acids, alcohols, diols, polyols, fatty acids, fatty acid alkyl esters (such as fatty acid methyl or ethyl esters (for example C6 to C12 fatty acid methyl esters (preferably C8 to C10 fatty acid methyl esters))), monacyl glycerides, diacyl glycerides, triacyl glycerides, and mixtures thereof. Preferred fermentation products are organic acids, amino acids, fatty acid alkyl esters (such as fatty acid methyl esters (for example C8 to C12 fatty acid methyl esters (preferably C8 to C10 fatty acid methyl esters))), and their salts thereof, and especially where the organic acid is selected from the group consisting of hydroxyl carboxylic acids (including mono-hydroxy and dihydroxy mono-, di-, and tri- carboxylic acids), monocarboxylic acids, dicarboxylic acids, and tricarboxylic acids and mixtures thereof. Examples of fermentation products that are prepared by the present process are organic acids or amino acids such as lactic acid, citric acid, malonic acid, hydroxy butyric acid, adipic acid, lysine, keto-glutaric acid, glutaric acid, 3-hydroxy-proprionic acid, succinic acid, malic acid, fumaric acid, itaconic acid, muconic acid, methacrylic acid, acetic acid, methyl hexanoate, methyl octanoate, methyl nonanoate, methyl decanoate, methyl dodecanoate, ethyl hexanoate, ethyl octanoate, ethyl nonanoate, ethyl decanoate, ethyl dodecanoate, and mixtures thereof and derivatives thereof and salts thereof. In a preferred aspect, a fermentation method of the disclosure produces ethanol as the bioproduct.

The fermentation product can have an excellent ratio of bioproduct (e.g., ethanol) to residual glucose, which is beneficial as it improves downstream processes, such as separation of components (e.g., ethanol) in the final fermentation medium. For example, the amount of glucose in the fermentation medium is 1.0 g/kg or less, 0.9 g/kg or less, 0.8 g/kg or less, 0.7 g/kg or less, 0.6 g/kg or less, 0.5 g/kg or less, 0.4 g/kg or less, 0.3 g/kg or less, or 0.2 g/kg or less, such as a glucose amount in the range of about 0.05 g/kg to about 1.0 g/kg, or about 0.05 g/kg to about 0.5 g/kg. The final fermentation medium can have an ethanol:glucose (wt/wt) ratio of about 70:1 (wt/wt) or greater, about 100:1 (wt/wt) or greater, about 150:1 (wt/wt) or greater, about 200:1 (wt/wt) or greater, about 250:1 (wt/wt) or greater, or about 300:1 (wt/wt)or greater, such as in the range of about 75:1 (wt/wt) to about 750:1 (wt/wt), or about 100:1 (wt/wt) to about 500:1 (wt/wt).

The fermentation product is recovered from the fermentation broth. The manner of accomplishing this will depend on the particular product. However, in some modes of practice, the organism is separated from the liquid phase, typically via a filtration step or centrifugation step, and the product recovered via, for example, distillation, extraction, crystallization, membrane separation, osmosis, reverse osmosis, or other suitable technique.

The present process provides the ability to make fermentation products on a production scale level with excellent yields and purity. In an aspect, the process is carried out in fermentation broth quantities of at least 25,000 gallons. In an aspect, the batch process is carried out in to produce batches of at least 25,000 gallons of final fermentation broth. In some aspects the process is a continuous process, performed in vessels of at least 200,000 gallons.

In some aspects, a genetically modified yeast expressing SEQ ID NO:1 or SEQ ID NO:4, or a variant thereof, can be used for conversion processes, such as for the production of dextrose for fructose syrups, specialty sugars and in alcohol and other end-product (e.g., organic acid, ascorbic acid, and amino acids). Production of alcohol from the fermentation of starch substrates using glucoamylases of the disclosure can include the production of fuel alcohol or potable alcohol.

Ethanol mass yield can be calculated by dividing the ethanol concentration by the total glucose consumed. Since glucose can be present as free glucose or tied up in oligomers, one needs to account for both. To determine the total glucose present at the beginning and end of fermentation, a total glucose equivalents measurement is determined. Total glucose equivalence measurement is as follows. Glucose is measured with HPLC using RI detection. Separation is completed with a Bio Rad 87H column using a 10 mM H2SO4 mobile phase. Glucose is measured in triplicate for each sample. An acid hydrolysis is performed in triplicate in 6% (v/v) trifluoroacetic acid at 121°C for 15 minutes. The resulting glucose after hydrolysis is measured by the same HPLC method. The total glucose equivalents present in each sample is the amount of glucose measured after acid hydrolysis. The total glucose consumed is calculated by subtracting the total glucose equivalents present at the end of fermentation from the total glucose equivalents present at the beginning of the fermentation.

Use of the engineered yeast of the current disclosure may also provide benefits with regards to increased titers, reduced volatile organic acids (VOCs), and reduced fusel oil compounds (volatile organic acids, higher alcohols, aldehydes, ketones, fatty acids and esters).

The fermentation product may be first treated with one or more agents via a treatment system. The treated fermentation product can then be sent to a distillation system. In the distillation system, the fermentation product can be distilled and dehydrated into ethanol. In some aspects, the components removed from the fermentation medium include water, soluble components, oil and unfermented solids. Some of these components can be used for other purposes, such as for an animal feed product. Other co-products, for example, syrup can be recovered from the stillage.

The present disclosure also provides a method for the production of a food, feed, or beverage product, such as an alcoholic or non-alcoholic beverage, such as a cereal- or malt-based beverage like beer or whiskey, such as wine, cider, vinegar, rice wine, soya sauce, or juice, said method comprising the step of treating a starch and/or sugar containing plant material with a composition as described herein. In another aspect, the disclosure also relates to a kit comprising a glucoamylase of the current disclosure, or a composition as contemplated herein; and instructions for use of said glucoamylase or composition. The disclosure also relates to a fermented beverage produced by a method using the glucoamylase.

After the fermentation process is complete, materials present in the fermentation medium can be of use. In some aspects, after a fermentation process has been completed, or while a fermentation process is ongoing, some or all of a bioproduct can be removed from the fermentation medium to provide a refined composition comprising non-bioproduct solids. The non-bioproduct solids can include the non-natural yeast, feedstock material in the medium that is not utilized by the yeast, as well as fermentation co-products. These materials can provide sources of carbohydrates and proteins that are useful as supplements to improve the nutritional content of a feed composition. The feed material can be a co-product from a fermentation process such as stillage (whole stillage, thin stillage, etc.) or composition prepared therefrom including dried distillers grains (DDG), distillers dry grains with solubles (DDGS), distillers wet grains (DWG), and distillers solubles (DS).

A fermentation medium, optionally with some or all of the target bioproduct removed, can be further treated, such as to remove water, or to cause precipitation or isolation of the non-bioproduct solids from the medium. In some cases the medium is treated by freeze drying or oven drying. After treatment the refined composition may be in the form of, for example, a liquid concentrate, a semi-wet cake, or a dry solid. The refined composition can be used as a feed composition itself, or an ingredient in the preparation of a feed composition. In preferred preparations, the feed composition is a livestock feed composition such as for sheep, cattle, pigs, etc.

The solids in the fermentation medium can provide a source of one or more amino acids. Introduced into an animal feed, the fermentation co-product can provide an enhanced amino acid content with regard to one or more essential amino acids. Essential amino acids can include histidine, isoleucine, lysine, methionine, phenylalanine, threonine, and tryptophan. These amino acids can be present in the feed composition as free amino acids or can be derived from proteins or peptides rich in the amino acids. The solids in the fermentation medium can provide a source of one or more prebiotics, which are nondigestible food substances, such as nondigestible oligosaccharides, that selectively stimulate the growth of favorable species of bacteria in the gut, thereby benefitting the host. The solids in the fermentation medium can provide a source of phytases, β-glucanases, proteases, and xylanases.

Table 4 includes strains used in the experimental studies associated with the disclosure.

**Table 4**

| **Strain ID** | **Strain Description** |
|---|---|
| Strain 1 | Wild Type |
| Strain 1-1 | Ura3Δ |
| Strain 1-2 | 2X Mfa2-*R*. *oryzae* GA |
| Strain 1-3 | 4X Mfa2-*R*. *oryzae* GA |
| Strain 1-4 | 1X *R. microsporus* GA |
| Strain 1-5 | 2X *R. microsporus* GA |
| Strain 1-6 | 2X *R. microsporus* GA |
| Strain 1-7 | 4X *R. microsporus* GA |
| Strain 1-8 | 2X *R. delemar* GA |
| Strain 1-9 | 4X *R. delemar* GA |

### Example 1

### Screening a diverse library of glucoamylase enzymes for growth on starch

Heterologous expression of a functional glucoamylase in *Saccharomyces cerevisiae* was first demonstrated circa 1993, using the *Aspergillus niger* glucoamylase. Other uses of glucoamylase in *Saccharomyces cerevisiae* have been reported, but still represent only a very small fraction of the number of public sequence information for these proteins. To that aim, over 1,000 enzymes were expressed and tested from a diverse set of organisms to identify enzymes that confer the desired trait of high glucoamylase expression while maintaining ethanol rate, titer, and yield.

A DNA library was constructed containing 1037 genes encoding glucoamylases, alpha-amylases, amylopullulanases, or other starch hydrolyzing enzymes by cloning synthetically created open reading frames into a *Saccharomyces cerevisiae* episomal plasmid. The enzymes encoded by these genes were sourced from four distinct classes including: 1) enzymes that were annotated with a glucoamylase EC number (which included both glucoamylases and a-amylases), 2) enzymes that were annotated as having both α-1,6 and α-1,4 glycosidase activity 3) structural homologs of previously identified functional fungal glucoamylases expressed in *Saccharomyces cerevisiae,* and finally 4) starch hydrolyzing enzymes from ruminant gut microbiomes. Each enzyme in the library was screened with its native leader as well as one substituted with the *Saccharomyces cerevisiae* Mfα2 leader. In total, 1,773 plasmids were successfully transformed into Strain 1-1 (described in previous application). Resulting transformants were tested for growth on starch containing media, using iodine staining to reveal zones of clearing. A total of 245 strains were able to produce zones of clearing, indicating that they contained plasmids with genes encoding heterologous enzymes capable of generating starch hydrolyzing activity when expressed in a yeast. These 245 were further screened for ethanol production as described below. The remaining genes encoded by the remaining 1528 plasmids were deemed not to be sufficiently active to warrant further testing.

### Secondary screening for ethanol production in deep well microtiters

Ethanol production was measured using deep well microtiter plates containing 0.5 mL of media. The fermentation medium consists of 725g partially hydrolyzed corn starch in the form of liquifact, 150g filtered light steep water, 125g sterile water, 25g glucose, and 1g urea. Partially hydrolyzed corn starch is provided by Cargill's Eddyville, Iowa corn wet mill (DS 30-37%, DE 5-15). Light steep water is also provided from Cargill's Eddyville, Iowa corn wet mill (free available nitrogen 2000-2500 ppm). Light steep water is centrifuged at 8,000 RPM, and the resulting supernatant is filter sterilized using 0.2 micron filters to produce filtered light steep water. Strains are inoculated to an OD₆₀₀ of 0.1 and the plate is incubated at 30°C with shaking in an orbital shake at 1000 rpm. Samples are taken and analyzed for relevant metabolite concentrations at the end of fermentation by HPLC.

Selected results from the screening of the 245 strains is shown in Table 5. Most of the strains did not demonstrate commercially relevant ethanol titers (e.g., *Aspergillus kawachii, Aspergillus terreus, Thermomyces lanuginosus,* and Mfα2*-Neurospora crassa* are representative of such strains). However, two strains (*Rhizopus delemar* and *Rhizopus microspores*) demonstrated ethanol titers greater than commercially relevant strains known in the art (Mfα2*-Rhizopus oryzae* and *Saccharomycopsis fibuligera*)*.*

**Table 5**

| **Enzyme Source, Accession #** | **SEQ ID NO** | **Ethanol titer (g/L) at 61.25 hours** |
|---|---|---|
| *Rhizopus delemar,* I1BGP8 | SEQ ID NO 7 | 100.08 |
| *Rhizopus microsporus,* A0A0C7BD37 | SEQ ID NO 8 | 107.82 |
| Mfα2*-Rhizopus oryzae,* Q2VC81 | SEQ ID NO 9 | 99.50 |
| *Saccharomycopsis fibuligera,* Q8TFE5 | SEQ ID NO 10 | 89.64 |
| *Aspergillus kawachii,* G7XVA6 | SEQ ID NO 11 | 33.70 |
| *Aspergillus terreus,* Q0CPK9 | SEQ ID NO 12 | 34.20 |
| *Thermomyces lanuginosus,* Q58HN1 | SEQ ID NO 13 | 35.00 |
| Mfα2*-Neurospora crassa,* A0A0B0E9D9 | SEQ ID NO 14 | 37.90 |

### Example 2

### Construction of strains expressing the MFalpha2-R. oryzae GA.

Creation of a ura3*Δ* auxotrophic base strain is previously described in (CAR0233P1 Strain 1-3), referred to as Strain 1-1 herein. Strain 1-1 is transformed with SEQ ID NO: 15 and SEQ ID NO: 16. SEQ ID NO: 15 contains the following elements: homology to integration locus A (3986bp), a ScTDH3 promoter (992-1673bp), a *Rhizopus oryzae* glucoamylase with modified signal sequence (1680-3476bp), a ScCYC1 terminator (3485-3708bp), a loxP recombination site (3717-3750bp), a ScURA3 promoter (3751-4257bp), the upstream portion of the ScURA3 (4258-4861bp). SEQ ID NO: 16 contains the following elements: downstream portion of the ScURA3 (7-606bp), a ScURA3 terminator (607-927bp), a loxP recombination site (928-961bp), a ScPGK1 promoter (968-1554bp), a *Rhizopus oryzae* glucoamylase with modified signal sequence (1561-3357bp), a ScGAL10 terminator (3366-3836bp), and homology to integration locus A (3838-4748bp). Transformants are selected on synthetic complete media lacking uracil. (ScD-Ura). Resulting transformants are streaked for single colony isolation on ScD-Ura. A single colony is selected. Correct integration of SEQ ID NO: 15 and SEQ ID NO: 16 into one allele of integration locus A is verified by PCR in the single colony. A PCR verified isolate is designated Strain 1-2 (yNS220).

Strain 1-2 is transformed with SEQ ID NO: 17, 18 and 19. SEQ ID NO: 17 contains the following elements: homology to integration locus A (3-986bp), a ScTDH3 promoter (9921673bp). SEQ ID NO: 18 contains the following elements: a ScTDH3 promoter (6-687bp), a *Rhizopus oryzae* glucoamylase with modified signal sequence (694-2490), a ScCYC1 terminator (2499-2722bp), a loxP recombination site (2731-2674bp), a ScTEF1 promoter (2765-3220bp), and the upstream portion of the *Aspergillus nidulans* acetamidase (3221-4260). SEQ ID NO: 19 contains the following elements: the downstream portion of the *Aspergillus nidulans* acetamidase (7-1032bp), a ScADH1 terminator (1033-1335bp), a loxP recombination site (1336-1369bp), a ScPGK1 promoter (1376-1962bp), a *Rhizopus oryzae* glucoamylase with modified signal sequence (1969-3765bp), a ScGAL10 terminator (3774-4244bp), and homology to integration locus A (4246-5008bp). Transformants are selected on Yeast Nitrogen Base (without ammonium sulfate or amino acids) containing 20 g/L glucose and 1g/L acetamide as the sole nitrogen source. Resulting transformants are streaked for single colony isolation on Yeast Nitrogen Base (without ammonium sulfate or amino acids) containing 20 g/l glucose and 1g/L acetamide as the sole nitrogen source. A single colony is selected. Correct integration of SEQ ID NO 17, 18 and 19 into the second allele of locus A is verified by PCR in the single colony. A PCR verified isolate is designated Strain 1-3.

### Example 3

### Construction of strains expressing the Rhizopus microsporus GA.

Strain 1-1 is transformed with SEQ ID NO: 20 and SEQ ID NO: 21. SEQ ID NO: 20 contains the following elements: homology to integration locus A (3-986bp), a ScTDH3 promoter (992-1673bp), a *Rhizopus microsporus* glucoamylase (1680-3497bp), a ScCYC1 terminator (3506-3729bp), a loxP recombination site (3738-3771bp), a ScURA3 promoter (3772-4278bp), the upstream portion of the ScURA3 (4279-4882bp). SEQ ID NO: 21 contains the following elements: A portion of the ScURA3 promoter (11-446), a ScURA3 (447-1250bp), a ScURA3 terminator (1251-1570bp), a loxP recombination site (1571-1604bp), and homology to integration locus A (1613-1790bp). Transformants are selected on synthetic complete media lacking uracil. (ScD-Ura). Resulting transformants are streaked for single colony isolation on ScD-Ura. A single colony is selected. Correct integration of SEQ ID NO: 20 and SEQ ID NO: 21into one allele of integration locus A is verified by PCR in the single colony. A PCR verified isolate is designated Strain 1-4.

Strain 1-4 is transformed with SEQ ID NO: 22 and SEQ ID NO: 23. SEQ ID NO: 22 contains the following elements: homology to integration locus A (1-193bp), a ScTDH3 promoter (199-880bp), a *Rhizopus microsporus* glucoamylase (887-2704bp), a ScCYC1 terminator (2713-2936bp), a loxP recombination site (2945-2978bp), a ScTEF1 promoter (2979-3434bp), and the upstream portion of the *Aspergillus nidulans* acetamidase (3435-4474bp). SEQ ID NO 23 contains the following elements: the downstream portion of the *Aspergillus nidulans* acetamidase (1-1498bp), a ScTEF1 terminator (1499-1658bp), a loxP recombination site (1692-1659bp), and homology to integration locus A (1701-1878). Transformants are selected on Yeast Nitrogen Base (without ammonium sulfate or amino acids) containing 20 g/L glucose and 1g/L acetamide as the sole nitrogen source. Resulting transformants are streaked for single colony isolation on Yeast Nitrogen Base (without ammonium sulfate or amino acids) containing 20 g/l glucose and 1g/L acetamide as the sole nitrogen source. A single colony is selected. Correct integration of SEQ ID NO: 22 and SEQ ID NO: 23 into the second allele of locus A is verified by PCR in the single colony. A PCR verified isolate is designated Strain 1-5.

Strain 1-1 is transformed with SEQ ID NO: 20 and SEQ ID NO: 24. SEQ ID NO: 24 contains the following elements: downstream portion of the ScURA3 (7-606bp), a ScURA3 terminator (607-927bp), a loxP recombination site (928-961bp), a ScPGK1 promoter (9681554bp), a Rhizopus microsporus glucoamylase (1561-3378bp), a ScGAL10 terminator (3387-3857bp), and homology to integration locus A (3859-4823bp). Transformants are selected on synthetic complete media lacking uracil. (ScD-Ura). Resulting transformants are streaked for single colony isolation on ScD-Ura. A single colony is selected. Correct integration of SEQ ID NO: 20 and SEQ ID NO: 24 into one allele of integration locus A is verified by PCR in the single colony. A PCR verified isolate is designated Strain 1-6.

Strain 1-6 is transformed with SEQ ID NO: 22 and SEQ ID NO: 25. SEQ ID NO: 25 contains the following elements: the downstream portion of the *Aspergillus nidulans* acetamidase (7-1032bp), a ScADH1 terminator (1033-1335bp), a loxP recombination site (1336-1369bp), a ScPGK1 promoter (1376-1962bp), a *Rhizopus microsporus* glucoamylase (1969-3786bp), a ScGAL10 terminator (3795-4265bp), and homology to integration locus A (4267-4684bp). Transformants are selected on Yeast Nitrogen Base (without ammonium sulfate or amino acids) containing 20 g/L glucose and 1g/L acetamide as the sole nitrogen source. Resulting transformants are streaked for single colony isolation on Yeast Nitrogen Base (without ammonium sulfate or amino acids) containing 20 g/l glucose and 1g/L acetamide as the sole nitrogen source. A single colony is selected. Correct integration of SEQ ID NO: 22 and SEQ ID NO: 25 into the second allele of locus A is verified by PCR in the single colony. A PCR verified isolate is designated Strain 1-7.

### Example 4

### Construction of strains expressing the Rhizopus delemar GA.

Strain 1-1 is transformed with SEQ ID NO: 26 and SEQ ID NO: 27. SEQ ID NO: 26 contains the following elements: homology to integration locus A (3-986bp), a ScTDH3 promoter (992-1673bp), a *Rhizopus delemar* glucoamylase (1698-3494bp), a ScCYC1 terminator (3503-3726bp), a loxP recombination site (3735-3768bp), a ScURA3 promoter (3769-4275bp), the upstream portion of the ScURA3 (4276-4879bp). SEQ ID NO: 27 contains the following elements: downstream portion of the ScURA3 (7-606bp), a ScURA3 terminator (607-927bp), a loxP recombination site (928-961bp), a ScPGK1 promoter (968-1554bp), a *Rhizopus delemar* glucoamylase (1561-3375bp), a ScGAL10 terminator (3384-3854bp), and homology to integration locus A (3856-4820). Transformants are selected on synthetic complete media lacking uracil. (ScD-Ura). Resulting transformants are streaked for single colony isolation on ScDUra. A single colony is selected. Correct integration of SEQ ID NO: 26 and SEQ ID NO: 27 into one allele of integration locus A is verified by PCR in the single colony. A PCR verified isolate is designated Strain 1-8.

Strain 1-8 is transformed with SEQ ID NO: 28 and SEQ ID NO: 29. SEQ ID NO: 28 contains the following elements: homology to integration locus A (1-986bp), a ScTDH3 promoter (992-1673bp), a *Rhizopus delemar* glucoamylase (1680-3494bp), a ScCYC1 terminator (3503-3726bp), a loxP recombination site (3735-3768bp), a ScTEF1 promoter (3769-4224bp), and the upstream portion of the *Aspergillus nidulans* acetamidase (4225-5264bp). SEQ ID NO: 29 contains the following elements: the downstream portion of the *Aspergillus nidulans* acetamidase (7-1032bp), a ScADH1 terminator (1033-1335bp), a loxP recombination site (1336-1369bp), a ScPGK1 promoter (1376-1962bp), a *Rhizopus delemar* glucoamylase (1969-3783bp), a ScGAL10 terminator (3792-4262bp), and homology to integration locus A (4264-5026bp). Transformants are selected on Yeast Nitrogen Base (without ammonium sulfate or amino acids) containing 20 g/L glucose and 1g/L acetamide as the sole nitrogen source. Resulting transformants are streaked for single colony isolation on Yeast Nitrogen Base (without ammonium sulfate or amino acids) containing 20 g/l glucose and 1g/L acetamide as the sole nitrogen source. A single colony is selected. Correct integration of SEQ ID NO: 28 and SEQ ID NO: 29 into the second allele of locus A is verified by PCR in the single colony. A PCR verified isolate is designated Strain 1-9.

### Example 5

### Characterization of strains in 32% DS corn mash at 30

### °C (TEST #1 and #2).

Strain 1, 1-3, 1-5, 1-7, 1-9 are struck to a YPD plate and incubated at 30°C until single colonies are visible (1-2 days). Cells from the YPD plate are scraped into pH 7.0 phosphate buffer and the optical density (OD600) is measured. Optical density is measured at wavelength of 600 nm with a 1 cm path length using a model Genesys20 spectrophotometer (Thermo Scientific). A shake flask is inoculated with the cell slurry to reach an initial OD600 of 0.1. Immediately prior to inoculating the following materials are added to each flask: 50 grams of liquified corn mash (32% DS, DE 30 +/- 2) is added to a 250 mL baffled shake flask sealed with air-lock containing 4mls of sterilized canola oil, 190ul of 500g/L filter-sterilized urea, and 2.5ul of 100mg/ml of filter sterilized ampicillin.

0.324 AGU/g DS (70µl of a 1:10 dilution) of glucoamylase (Spirizyme Fuel HS, Novozymes) is added to flasks containing the control Strain 1, and either zero or 0.097 AGU/g DS (21 µL of a 1:10 dilution of glucoamylase (Spirizyme Fuel HS^{™}, Novozymes is added to the remaining flasks depending on the "Test". Spirizyme Fuel HS^{™} is estimated to have approximately 769 AGU/g enzyme, however over time the activity can change 10-20% (i.e., the activity of the enzyme typically decreases over time). Duplicate flasks for each strain are incubated at 30°C with shaking in an orbital shaker at 100 rpm for approximately 48 hours. At 48 hours, 1ml samples are taken and analyzed for ethanol and glucose concentrations in the broth by high performance liquid chromatography with refractive index detector. Selected results are shown in Table 6.

**Table 6**

| | Test #1 Final EtOH titer (g/kg) | Test #1 Residual Glucose (g/kg) |
|---|---|---|
| Strain 1, with 0.324 AGU/g DS | 133.6 +/- 1.6 | 0.4 +/- 0.1 |
| Strain 1-3, no supplementation | 132.5 +/- 0.3 | 0.2 +/- 0.0 |
| Strain 1-5, no supplementation | 120.9 +/- 1.3 | 0.5 +/- 0.1 |
| Strain 1-7, no supplementation | 134.1 +/- 4.8 | 0.2 +/- 0.0 |
| Strain 1-9, no supplementation | 119.8 +/- 1.9 | 0.9 +/- 0.0 |
| | | |

| | Test #2 Final EtOH titer (g/kg) | Test #2 Residual Glucose (g/kg) |
|---|---|---|
| Strain 1, with 0.324 AGU/g DS | 133.6 +/- 1.6 | 0.4 +/- 0.1 |
| Strain 1-3, no supplementation | 130.7 +/- 2.7 | 0.2 +/- 0.1 |
| Strain 1-5, no supplementation | 132.9 +/- 0.2 | 0.3 +/- 0.0 |
| Strain 1-7, no supplementation | 135.0 +/- 1.2 | 0.3 +/- 0.0 |
| Strain 1-9, no supplementation | 131.9 +/- 0.5 | 0.2 +/- 0.1 |

### Example 6

### Characterization of strains in 32% DS corn mash at 33.3°C (TEST #3 and #4).

Strain 1, 1-3, 1-5, 1-7, 1-9 are struck to a YPD plate and incubated at 30°C until single colonies are visible (1-2 days). Cells from the YPD plate are scraped into pH 7.0 phosphate buffer and the optical density (OD₆₀₀) is measured. Optical density is measured at wavelength of 600 nm with a 1 cm path length using a model Genesys20 spectrophotometer (Thermo Scientific). A shake flask is inoculated with the cell slurry to reach an initial OD₆₀₀ of 0.1. Immediately prior to inoculating the following materials are added to each flask: 50 grams of liquified corn mash is added to a 250 mL baffled shake flask sealed with air-lock containing 4mls of sterilized canola oil, 190ul of 500g/L filter-sterilized urea, and 2.5ul of 100mg/ml of filter sterilized ampicillin. 0.324 AGU/g DS (70µl of a 1:10 dilution) of glucoamylase (Spirizyme Fuel HS^{™}, Novozymes) is added to flasks containing the control Strain 1, and either zero or 0.097 AGU/g DS (21µl of a 1:10 dilution) of glucoamylase (Spirizyme Fuel HS^{™}, Novozymes) is added to the remaining flasks, depending on the "Test". Spirizyme Fuel HS^{™} is estimated to have approximately 326 AGU/g enzyme. Duplicate flasks for each strain are incubated at 33.3°C with shaking in an orbital shake at 100 rpm for approximately 48 hours. At 48 hours, 1ml samples are taken and analyzed for ethanol and glucose concentrations in the broth by high performance liquid chromatography with refractive index detector. Selected results are shown in Table 7.

**Table 7**

| | Test #3 Final EtOH titer (g/kg) | Test #3 Residual Glucose (g/kg) |
|---|---|---|
| Strain 1, with 0.324 AGU/g DS | 135.8 +/- 0.4 | 0.8 +/- 0.1 |
| Strain 1-3, no supplementation | 124.1 +/- 4.4 | 6.0 +/- 0.0 |
| Strain 1-5, no supplementation | 132.8 +/- 1.2 | 0.2 +/- 0.0 |
| Strain 1-7, no supplementation | 131.2 +/- 2.4 | 2.6 +/- 0.2 |
| Strain 1-9, no supplementation | 132.9 +/- 1.2 | 0.2 +/- 0.0 |
| | | |

| | Test #4 Final EtOH titer (g/kg) | Test #4 Residual Glucose (g/kg) |
|---|---|---|
| Strain 1, with 0.324 AGU/g DS | 135.8 +/- 0.4 | 0.8 +/- 0.1 |
| Strain 1-3 with 0.097 AGU/g DS | 129.5 +/- 3.6 | 3.3 +/- 0.0 |
| Strain 1-5, with 0.097 AGU/g DS | 132.8 +/- 0.3 | 0.4 +/- 0.2 |
| Strain 1-7, with 0.097 AGU/g DS | 129.5 +/- 0.9 | 7.9 +/- 0.2 |
| Strain 1-9, with 0.097 AGU/g DS | 134.5 +/- 1.5 | 0.9 +/- 0.1 |

### Example 7

### Characterization of strains in 34% DS corn mash at 33.3°C.

Strains 1, 1-3, 1-6, and 1-9 are struck to a YPD plate and incubated at 30°C until single colonies are visible (1-2 days). Cells from the YPD plate are scraped into pH 7.0 phosphate buffer and the optical density (OD₆₀₀) is measured. Optical density is measured at wavelength of 600 nm with a 1 cm path length using a model Genesys20 spectrophotometer (Thermo Scientific). A shake flask is inoculated with the cell slurry to reach an initial OD₆₀₀ of 0.1. Immediately prior to inoculating the following materials are added to each flask: 50 grams of liquified corn mash is added to a 250 mL baffled shake flask sealed with air-lock containing 4mls of sterilized canola oil, 190ul of 500g/L filter-sterilized urea, and 2.5ul of 100mg/ml of filter sterilized ampicillin. 0.324 AGU/g DS (70µl of a 1:10 dilution) of glucoamylase (Spirizyme Fuel HS^{™}, Novozymes) is added to flasks containing the control Strain 1, and either zero or 0.032 AGU/g DS, 0.065 AGU/g DS, 0.097 AGU/g DS, or 0.162 AGU/gDS. (7 µl, 14 µl, 21µl, or 35 µl of a 1:10 dilution) is added to the remaining flasks. Spirizyme Fuel HS^{™} is estimated to have approximately 326 AGU/g enzyme. Duplicate flasks for each strain are incubated at 33.3°C with shaking in an orbital shake at 100 rpm for approximately 48 hours. At various intervals, the flasks are opened and samples are analyzed for ethanol and glucose concentrations in the broth by high performance liquid chromatography with refractive index detector. Selected results are shown in Table 8.

**Table 8**

| | Final EtOH titer (g/kg) | Residual Glucose (g/kg) |
|---|---|---|
| Strain 1, 0.324 AGU/g DS dose | 137.0 +/- 2.8 | 1.2 +/- 0.3 |
| Strain 1-3, no supplementation | 123.2 +/- 1.1 | 12.1 +/- 0.5 |
| Strain 1-3, 0.032 AGU/g DS dose | 122.3 +/- 3.2 | 14.8 +/- 0.8 |
| Strain 1-3, 0.065 AGU/g DS dose | 123.4 +/- 4.2 | 16.8 +/- 0.0 |
| Strain 1-3, 0.097 AGU/g dose DS | 130.1 +/- 1.4 | 14.6 +/- 0.3 |
| Strain 1-6, no GA dose | 133.9 +/- 5.1 | 0.2 +/- 0.1 |
| Strain 1-6,0.032 AGU/g dose DS | 137.5 +/- 3.7 | 0.6 +/- 0.0 |
| Strain 1-6,0.097 AGU/g dose DS | 138.8 +/- 0.2 | 1.0 +/- 0.0 |
| Strain 1-6, 0.162AGU/gDS dose | 138.4 +/- 2.8 | 2.0 +/- 0.7 |
| Strain 1-9, no supplementation | 135.1 +/- 0.3 | 1.2 +/- 0.1 |
| Strain 1-9, 0.032 AGU/g DS dose | 136.2 +/- 5.4 | 1.4 +/- 0.2 |
| Strain 1-9,0.097 AGU/g DS dose | 135.7 +/- 1.0 | 4.8 +/- 0.9 |
| Strain 1-9, 0.162 AGU/gDS dose | 136.0 +/- 2.0 | 6.1 +/- 0.3 |

### Example 8

### Characterization of a strain in 32% DS corn mash having a DE of 30 +/- 2 at a temperature of 30°C.

A strain is struck to a YPD plate (20g/L yeast peptone, 10g/L yeast extract, 20g/L glucose, and 20g/L agar) and incubated at 30°C until single colonies are visible (1-2 days). Cells from the YPD plate are scraped into pH 7.0 phosphate buffer to create a cell slurry and the optical density (OD₆₀₀) is measured. Optical density is measured at wavelength of 600 nm with a 1 cm path length using a model Genesys20 spectrophotometer (Thermo Scientific). A shake flask is inoculated with the cell slurry to reach an initial OD₆₀₀ of 0.1. Immediately prior to inoculating the following materials are added to each flask: 50 grams of liquified corn mash (32% DS, DE 30) is added to a 250 mL baffled shake flask sealed with air-lock containing 4mls of sterilized canola oil, 190ul of 500g/L filter-sterilized urea, and 2.5ul of 100mg/ml of filter sterilized ampicillin. The shake flask, airlock, canola oil is weighed prior to the addition of the fermentation media and cells, which is subtracted from the total weight of the flask to give the starting media volume. At various time points in the fermentation, the flasks are removed and the weight recorded. The mass loss (grams) at any timepoint is calculated by subtracting the mass at T1 from the original mass at T0. The mass loss (grams) is converted to a mass loss (percentage) by dividing the mass loss at any given time point by the original starting mass. The percentage mass loss can be converted to g/kg ethanol by the following equation (the starting mass of the fermentation media. Ethanol (g/kg) = (Percent mass loss + 0.0016)/0.0009/1.042.

The engineered yeast of the invention may produce a greater amount of ethanol than a parent strain that does not include the exogenous nucleic acid under the same fermentation conditions.

In the fermentation method of the disclosure, the fermentation medium, at the beginning of fermentation, may have a glucose concentration of about 30 g/kg or less.

## Claims

1. An engineered yeast comprising an exogenous nucleic acid encoding a glucoamylase comprising a sequence having 81% or greater sequence identity to SEQ ID NO:1, wherein the yeast is capable of producing ethanol at a rate of 1 g/L*h or greater during a fermentation process,
wherein there are 2 - 8 copies of the exogenous nucleic acid in the cell.

2. An engineered yeast comprising an exogenous nucleic acid encoding a glucoamylase comprising a sequence having 81% or greater sequence identity to SEQ ID NO:1, wherein the yeast is capable of producing ethanol at a rate of 1 g/L*h or greater during a fermentation process,
wherein the genetically engineered yeast does not express a heterologous trehalase.

3. The engineered yeast of claim 1, wherein the yeast is capable of producing (a) at least 70g/kg of ethanol in a fermentation medium made from a glucose polymer-containing feedstock having (i) a dextrose equivalent, DE, of not greater than 50.

4. The engineered yeast of claim 1, wherein the yeast is capable of producing (a) at least 70g/kg of ethanol in a fermentation medium made from corn mash having a dextrose equivalent, DE, of 30 +/- 2, wherein the fermentation medium comprises 32% dry wt corn, and a pH 5.8, 35 ppm CaCl, 1900 ppm urea, 5 ppm ampicillin, wherein the starting yeast concentration is 0.1 (OD600), and fermentation is carried out at 48 hrs at 30°C with agitation.

5. The engineered yeast of any of claims 1, 3 or 4, wherein the glucoamylase comprises a sequence having 85% or greater sequence identity to SEQ ID NO:1.

6. The engineered yeast of claim 5, wherein the glucoamylase comprises a sequence having 90% or greater sequence identity to SEQ ID NO:1.

7. The engineered yeast of claim 6, wherein the glucoamylase comprises a sequence having 95% or greater sequence identity to SEQ ID NO:1.

8. The engineered yeast of any of claims 1 or 3 to 7 which is a *Saccharomyces cerevisiae* yeast.

9. The engineered yeast of any of claims 1 or 3 to 8 which is tolerant to growth in a fermentation medium having a concentration of ethanol of greater than 90 g/L.

10. The engineered yeast of any of claims 1 or 3 to 9 which is tolerant to growth at temperatures in the range of greater than 31°C - 35 °C.

11. A fermentation method for producing a bioproduct, comprising: forming a fermentation medium from a glucose polymer-containing feedstock; and fermenting the fermentation medium using an engineered yeast comprising an exogenous nucleic acid encoding a glucoamylase comprising a sequence having 81% or greater sequence identity to SEQ ID NO:1, wherein fermenting produces a bioproduct,
wherein there are 2 - 8 copies of the exogenous nucleic acid in the cell.

12. The fermentation method of claim 11, wherein the glucose polymer-containing feedstock or the fermentation medium, at the beginning of fermentation, has a dextrose equivalent, DE, of about 50 or less.

13. The fermentation method of claims 11 or 12 wherein the glucose polymer-containing feedstock comprises glucose polymer having a degree of polymerization of 4 or greater and present in an amount of 75% weight or greater total fermentable carbohydrates in the feedstock.

14. The fermentation of any of claims 11-13 wherein ethanol is produced to a concentration of 70 g/L or greater in the medium.

15. The fermentation method of any of claims 11-14 comprising adding supplemental glucoamylase to the feedstock, or supplemental glucoamylase to the medium during the fermentation period.

## Patentansprüche

1. Manipulierte Hefe, umfassend eine exogene Nucleinsäure, die für eine Glucoamylase codiert, umfassend eine Sequenz, die eine Sequenzidentität zu SEQ ID NO:1 von 81 % oder mehr aufweist, wobei die Hefe in der Lage ist, während eines Fermentationsprozesses Ethanol mit einer Rate von 1 g/L*h oder mehr zu produzieren,
wobei 2-8 Kopien der exogenen Nucleinsäure in der Zelle vorliegen.

2. Manipulierte Hefe, umfassend eine exogene Nucleinsäure, die für eine Glucoamylase codiert, umfassend eine Sequenz, die eine Sequenzidentität zu SEQ ID NO:1 von 81 % oder mehr aufweist, wobei die Hefe in der Lage ist, während eines Fermentationsprozesses Ethanol mit einer Rate von 1 g/L*h oder mehr zu produzieren,
wobei die genmanipulierte Hefe keine heterologe Trehalase exprimiert.

3. Manipulierte Hefe nach Anspruch 1, wobei die Hefe in der Lage ist, (a) mindestens 70 g/kg Ethanol in einem Fermentationsmedium zu produzieren, das aus einem glucosepolymerhaltigen Rohstoff, der (i) ein Dextroseäquivalent, DE, von nicht mehr als 50 aufweist, hergestellt ist.

4. Manipulierte Hefe nach Anspruch 1, wobei die Hefe in der Lage ist, (a) mindestens 70 g/kg Ethanol in einem Fermentationsmedium zu produzieren, das aus Maismaische, die ein Dextroseäquivalent, DE, von 30 +/- 2 aufweist, hergestellt ist, wobei das Fermentationsmedium zu 32 % Trockengewicht Mais und einen pH-Wert von 5,8, 35 ppm CaCl, 1900 ppm Harnstoff, 5 ppm Ampicillin umfasst, wobei die Hefeausgangskonzentration 0,1 (OD600) beträgt und eine Fermentation für 48 Stunden bei 30 °C unter Rühren durchgeführt wird.

5. Manipulierte Hefe nach einem der Ansprüche 1, 3 oder 4, wobei die Glucoamylase eine Sequenz, die eine Sequenzidentität zu SEQ ID NO:1 von 85 % oder mehr aufweist, umfasst.

6. Manipulierte Hefe nach Anspruch 5, wobei die Glucoamylase eine Sequenz, die eine Sequenzidentität zu SEQ ID NO:1 von 90 % oder mehr aufweist, umfasst.

7. Manipulierte Hefe nach Anspruch 6, wobei die Glucoamylase eine Sequenz, die eine Sequenzidentität zu SEQ ID NO:1 von 95 % oder mehr aufweist, umfasst.

8. Manipulierte Hefe nach einem der Ansprüche 1 oder 3 bis 7, die eine Hefe *Saccharomyces cerevisiae* ist.

9. Manipulierte Hefe nach einem der Ansprüche 1 oder 3 bis 8, die tolerant gegenüber Wachstum in einem Fermentationsmedium, das eine Ethanolkonzentration von mehr als 90 g/L aufweist, ist.

10. Manipulierte Hefe nach einem der Ansprüche 1 oder 3 bis 9, die tolerant gegenüber Wachstum bei Temperaturen in dem Bereich von mehr als 31 °C-35 °C ist.

11. Fermentationsverfahren zum Produzieren eines Bioprodukts, umfassend: Ausbilden eines Fermentationsmediums aus einem glucosepolymerhaltigen Rohstoff; und Fermentieren des Fermentationsmediums unter Verwendung einer manipulierten Hefe, umfassend eine exogene Nucleinsäure, die für eine Glucoamylase codiert, umfassend eine Sequenz, die eine Sequenzidentität zu SEQ ID NO:1 von 81 % oder mehr aufweist, wobei das Fermentieren ein Bioprodukt produziert,
wobei 2-8 Kopien der exogenen Nucleinsäure in der Zelle vorliegen.

12. Fermentationsverfahren nach Anspruch 11, wobei der glucosepolymerhaltige Rohstoff oder das Fermentationsmedium, zu Beginn der Fermentation, ein Dextroseäquivalent, DE, von etwa 50 oder weniger aufweist.

13. Fermentationsverfahren nach Anspruch 11 oder 12, wobei der glucosepolymerhaltige Rohstoff Glucosepolymer, das einen Polymerisationsgrad von 4 oder mehr aufweist und in einer Menge von zu 75 Gew.-% oder mehr an gesamten fermentierbaren Kohlenhydraten in dem Rohstoff vorhanden ist, umfasst.

14. Fermentation nach einem der Ansprüche 11 bis 13, wobei Ethanol auf eine Konzentration von 70 g/L oder mehr in dem Medium produziert wird.

15. Fermentationsverfahren nach einem der Ansprüche 11 bis 14, umfassend ein Zugeben von zusätzlicher Glucoamylase zu dem Rohstoff oder von zusätzlicher Glucoamylase zu dem Medium während des Fermentationszeitraums.

## Revendications

1. Levure modifiée comprenant un acide nucléique exogène codant pour une glucoamylase comprenant une séquence ayant une identité de séquence de 81 % ou plus avec SEQ ID NO: 1, dans laquelle la levure est capable de produire de l'éthanol à une vitesse de 1 g/L*h ou plus pendant un processus de fermentation,
dans laquelle il y a 2 à 8 copies de l'acide nucléique exogène dans la cellule.

2. Levure modifiée comprenant un acide nucléique exogène codant pour une glucoamylase comprenant une séquence ayant une identité de séquence de 81 % ou plus avec SEQ ID NO: 1, dans laquelle la levure est capable de produire de l'éthanol à une vitesse de 1 g/L*h ou plus pendant un processus de fermentation,
dans laquelle la levure génétiquement modifiée n'exprime pas de tréhalase hétérologue.

3. Levure modifiée selon la revendication 1, dans laquelle la levure est capable de produire (a) au moins 70 g/kg d'éthanol dans un milieu de fermentation préparé à partir d'une charge d'alimentation contenant un polymère de glucose ayant (i) un équivalent dextrose, DE, non supérieur à 50.

4. Levure modifiée selon la revendication 1, dans laquelle la levure est capable de produire (a) au moins 70 g/kg d'éthanol dans un milieu de fermentation préparé à partir de moût de maïs ayant un équivalent dextrose, DE, de 30 +/- 2, dans laquelle le milieu de fermentation comprend 32 % en poids sec de maïs, et un pH de 5,8, 35 ppm de CaCl, 1 900 ppm d'urée, 5 ppm d'ampicilline, dans laquelle la concentration de levure de départ est de 0,1 (DO600), et la fermentation est effectuée pendant 48 heures à 30 °C avec agitation.

5. Levure modifiée selon l'une quelconque des revendications 1, 3 ou 4, dans laquelle la glucoamylase comprend une séquence ayant une identité de séquence de 85 % ou plus avec SEQ ID NO: 1.

6. Levure modifiée selon la revendication 5, dans laquelle la glucoamylase comprend une séquence ayant une identité de séquence de 90 % ou plus avec SEQ ID NO: 1.

7. Levure modifiée selon la revendication 6, dans laquelle la glucoamylase comprend une séquence ayant une identité de séquence de 95 % ou plus avec SEQ ID NO: 1.

8. Levure modifiée selon l'une quelconque des revendications 1 ou 3 à 7, qui est une levure *Saccharomyces cerevisiae*.

9. Levure modifiée selon l'une quelconque des revendications 1 ou 3 à 8, qui est tolérante à la croissance dans un milieu de fermentation ayant une concentration en éthanol supérieure à 90 g/L.

10. Levure modifiée selon l'une quelconque des revendications 1 ou 3 à 9, qui est tolérante à la croissance à des températures comprises dans la plage de plus de 31 °C - 35 °C.

11. Procédé de fermentation permettant de produire un bioproduit, comprenant : la formation d'un milieu de fermentation à partir d'une charge d'alimentation contenant un polymère de glucose ; et la fermentation du milieu de fermentation à l'aide d'une levure modifiée comprenant un acide nucléique exogène codant pour une glucoamylase comprenant une séquence ayant une identité de séquence de 81 % ou plus avec SEQ ID NO: 1, dans lequel la fermentation produit un bioproduit,
dans lequel il y a 2 à 8 copies de l'acide nucléique exogène dans la cellule.

12. Procédé de fermentation selon la revendication 11, dans lequel la charge d'alimentation contenant un polymère de glucose ou le milieu de fermentation, au début de la fermentation, a un équivalent dextrose, DE, d'environ 50 ou moins.

13. Procédé de fermentation selon les revendications 11 ou 12, dans lequel la charge d'alimentation contenant un polymère de glucose comprend un polymère de glucose ayant un degré de polymérisation de 4 ou plus et présent en une quantité de 75 % en poids ou plus des glucides fermentescibles totaux dans la charge d'alimentation.

14. Fermentation selon l'une quelconque des revendications 11 à 13, dans laquelle de l'éthanol est produit à une concentration de 70 g/L ou plus dans le milieu.

15. Procédé de fermentation selon l'une quelconque des revendications 11 à 14, comprenant l'ajout de glucoamylase supplémentaire à la charge d'alimentation, ou de glucoamylase supplémentaire au milieu pendant la période de fermentation.
